# EUROPEAN PATENT APPLICATION

(11) **EP 3 686 275 A1**
(43) Date of publication of application: **29.07.2020**
(21) Application number: 18857205.1
(22) Date of filing: 18.09.2018
(51) Int. Cl.: C12N 5/10

(54) **GENE EDITING T CELL AND USE THEREOF**

(30) Priority: 18.09.2017 CN 201710842264; 18.09.2017 CN 201710841323
(71) Applicant: Edigene Inc., Beijing 102206 (CN)
(72) Inventor: YUAN, Pengfei, Beijing 102206 (CN); WANG, Fei, Beijing 102206 (CN); YU, Lingling, Beijing 102206 (CN); DONG, Xi, Beijing 102206 (CN)
(74) Representative: Lavoix
(86) International application number: PCT/CN2018/106237
(87) International publication number: WO 2019/052577

(57) **Abstract**

Provided are a universal T cell and preparation method thereof. The universal T cells comprise CAR-T and TCR-T cells, and are obtained by knocking out TCR and/or HLA and/or PD-1 proteins of T cells by a CRISPR/Cas9 genome editing technology, wherein the universal CAR-T continues to kill target cells *in vivo* and *in vitro.*

## Description

### CROSS REFERENCE

This application claims the priorities of Chinese patent applications CN201710842264.5 and CN201710841323.7 filed on September 18, 2017, each disclosure of them is incorporated herein by reference in its entirety.

### FIELD OF THE INVENTION

The present invention relates to a T cell, and in particular to a single-gene, double-genes or triple-genes knockout genetically modified cell, comprising a universal T cell, a CAR-T cell and a TCR-T cell, and preparation method and application thereof.

### BACKGROUND OF THE INVENTION

Malignant tumors have become a serious threat to health and life, and the cure of cancer has always been a human dream. In recent years, from the first application of CAR-T technique in 1989, to the cure of Emily Whitehead by a team led by Professor Carl June of the University of Pennsylvania in 2012, and to the recommendation of Novartis CAR-T drug CTL019 for the treatment of adolescent advanced B-cell acute lymphoblastic leukemia (r/rAll) was approved by a 10:0 overwhelming vote by the FDA Oncologic Drugs Advisory Committee (ODAC) in 2017, tumor immunotherapy, especially CAR-T (Chimeric Antigen Receptor T Cells) technique, the emergence of which has led to a milestone in the development of the control of tumors, has attracted extensive attention.

In general, the T cells used in traditional CAR-T technique are mainly derived from the patients themselves. The T cells are isolated, activated, CAR introduced, cultured and expanded in a GMP environment in vitro, and finally infused back to the patient after quality control. Problems emerge due to patient's own conditions. For example, some of them are unsuitable for blood collection, or their T cells are difficult in proliferation after being isolated from the collected blood. When the patient is in critical condition, the waiting time of the whole process from the isolation of T cells to the infusion of CAR-T will also be a major problem for autologous infusion. These problems have brought limitations to the extensive use of CAR-T technique. Therefore, an important research direction of CAR-T cell therapy is the method to prepare a large number of CAR-T cells by using T cells of a healthy blood donor so as to meet the clinical needs of patients. The establishment of this technique will greatly reduce the cost of CAR-T therapy, thereby better guaranteeing the quality of the uniformly prepared cells. At the same time, patients can get CAR-T cells for treatment whenever needed.

Several documents are cited throughout the text of this specification. Each document (including any journal articles or abstracts, published or unpublished patent applications, authorized patents, manufacturer's specifications, instructions for use, etc.) is incorporated herein by reference in its entirety. However, it is not an admission that the documents cited herein are in fact the prior art to the present invention.

### SUMMARY OF THE INVENTION

The present invention aims to solve the above problems in the art. The invention performs single-gene (TRAC, B2M or PD-1), double-gene (TRAC and B2M) and triple-gene (TRAC, B2M and PD-1) knockout in a T cell by CRISPR/Cas9 system, and the knockout efficiency is respectively up to 90% (single gene), 81% (double genes) and 67% (triple genes). These genetically modified T cells can be provided as universal T cells for CAR or TCR targeting different targets. Provided are methods for establishing the treatment for tumors and viral infectious diseases (e.g., HIV/AIDS) by using genome editing technology in conjunction with adoptive immunization, laying a solid technical foundation for the research on the treatment of related diseases. At the same time, the genetically modified T cells (including universal T cells, CAR-T and TCR-T) are capable to be used as a drug at any time for patients in need.

Thus, in one aspect, the present invention provides a method for obtaining a universal T cell not expressing TCR, or TCR/HLA, or TCR/HLA/PD-1 by efficient knockout of single-gene (TRAC, B2M or PD-1), double-gene (TRAC and B2M) or triple-gene (TRAC, B2M, and PD-1) from the T cell, using genome editing technology such as the CRISPR/Cas9 system. At the same time, the universal T cell together with the required CAR or TCR can be made into a universal CAR-T or TCR-T as a medicine for a patient in need at any time. Additionally, it also supports the research on new and effective gene targets, and at the same time it is applied to clinical immunotherapy in a timely and effective manner.

In one aspect, the invention provides a method for preparing a genetically modified T cell, which comprises disrupting the following genomic regions in the T cell by genome editing technology:
(i) the TRAC genomic region from base 23016448 to 23016490 on chromosome 14 (represented by SEQ ID NO: 23);
(ii) the B2M genomic region from base 45003745 to 45003788 on chromosome 15 (represented by SEQ ID NO: 24); and/or
(iii) the PD-1 genomic region from base 242800936 to 242800978 on chromosome 2 (represented by SEQ ID NO: 25), or the PD-1 genomic region from base 242795009 to 242795051 on chromosome 2 (represented by SEQ ID NO: 26). In some embodiments, all of the TRAC genomic region, the B2M genomic region, and the PD-1 genomic region are edited. In some embodiments, the genome editing technology is a zinc finger nuclease-based genome editing technology, a TALEN genome editing technology, or a CRISPR/Cas genome editing technology, such as a CRISPR/Cas9 genome editing technology.

In some embodiments, the invention provides a method for preparing a genetically modified T cell, which comprises disrupting the following target nucleotide sequences in the T cell by genome editing technology:
(i) a target nucleotide sequence of the TRAC gene complementary to a sequence selected from any one of SEQ ID NOs: 2-5;
(ii) a target nucleotide sequence of the B2M gene complementary to a sequence selected from any one of SEQ ID NOs: 6-13; and/or
(iii) a target nucleotide sequence of the PD-1 gene complementary to a sequence selected from any one of SEQ ID NOs: 14-22. In some embodiments, all of the TRAC genomic region, the B2M genomic region, and the PD-1 genomic region are edited. In some embodiments, the genome editing technology is a zinc finger nuclease-based genome editing technology, a TALEN genome editing technology, or a CRISPR/Cas genome editing technology, such as a CRISPR/Cas9 genome editing technology.

In some embodiments, the invention provides a method for preparing a genetically modified T cell, which comprises disrupting the following target nucleotide sequences in the T cell by a CRISPR/Cas9 genome editing technology:
(i) a target nucleotide sequence of the TRAC gene complementary to a sequence selected from any one of SEQ ID NO: 2 or SEQ ID NO: 3;
(ii) a target nucleotide sequence of the B2M gene complementary to a sequence selected from any one of SEQ ID NO: 7 or SEQ ID NO: 8;
(iii) a target nucleotide sequence of the PD-1 gene complementary to a sequence selected from any one of SEQ ID NO: 14 or SEQ ID NO: 15.

In some embodiments, the invention provides a method for preparing a genetically modified T cell, which comprises disrupting the following target nucleotide sequences in the T cell by a CRISPR/Cas9 genome editing technology:
(i) a target nucleotide sequence of the TRAC gene complementary to the sequence of SEQ ID NO: 2;
(ii) a target nucleotide sequence of the B2M gene complementary to the sequence of SEQ ID NO: 8; and/or
(iii) a target nucleotide sequence of the PD-1 gene complementary to the sequence of SEQ ID NO: 14.

In some embodiments, the invention provides a method for preparing a genetically modified T cell, which comprises disrupting the following target nucleotide sequences in the T cell by a CRISPR/Cas9 genome editing technology:
(i) a target nucleotide sequence of the TRAC gene complementary to the sequence of SEQ ID NO: 2;
(ii) a target nucleotide sequence of the B2M gene complementary to the sequence of SEQ ID NO: 8; and/or
(iii) a target nucleotide sequence of the PD-1 gene complementary to the sequence of SEQ ID NO: 15.

In some embodiments, the invention provides a method for preparing a genetically modified T cell, which comprises disrupting the following target nucleotide sequences in the T cell by a CRISPR/Cas9 genome editing technology:
(i) a target nucleotide sequence of the TRAC gene complementary to the sequence of SEQ ID NO: 3;
(ii) a target nucleotide sequence of the B2M gene complementary to the sequence of SEQ ID NO: 7; and/or
(iii) a target nucleotide sequence of the PD-1 gene complementary to the sequence of SEQ ID NO: 15.

In some embodiments, the invention provides a method for preparing a genetically modified T cell by a CRISPR/Cas9 genome editing technology, wherein:
(i) introducing a sgRNA comprising a sequence selected from any one of SEQ ID NOs: 2-5 into the T cell for editing a TRAC genomic region from base 23016448 to 23016490 on chromosome 14;
(ii) introducing a sgRNA comprising a sequence selected from any one of SEQ ID NOs: 6-13 into the T cell for editing the B2M genomic region from base 45003745 to 45003788 on chromosome 15; and/or
(iii) introducing a sgRNA comprising a sequence selected from any one of SEQ ID NOs: 14-22 into the T cell for editing the PD-1 genomic region from base 242800936 to 242800978 on chromosome 2, or the PD-1 genomic region from base 242795009 to 242795051 on chromosome 2.

In some embodiments, the invention provides a method for preparing a genetically modified T cell by a CRISPR/Cas9 genome editing technology, wherein:
(i) introducing a sgRNA comprising a sequence selected from any one of SEQ ID NO: 2 or SEQ ID NO: 3 into the T cell for editing the TRAC genomic region from base 23016448 to 23016490 on chromosome 14;
(ii) introducing a sgRNA comprising a sequence selected from any one of SEQ ID NO: 7 or SEQ ID NO: 8 into the T cell for editing the B2M genomic region from base 45003745 to 45003788 on chromosome 15; and/or
(iii) introducing a sgRNA comprising a sequence selected from any one of SEQ ID NO: 14 or SEQ ID NO: 15 into the T cell for editing the PD-1 genomic region from base 242800936 to 242800978 on chromosome 2, or the PD-1 genomic region from base 242795009 to 242795051 on chromosome 2.

In some embodiments, the invention provides a method for preparing a genetically modified T cell by a CRISPR/Cas9 genome editing technology, wherein:
(i) introducing a sgRNA comprising the sequence of SEQ ID NO: 2 into the T cell for editing the TRAC genomic region from base 23016448 to 23016490 on chromosome 14;
(ii) introducing a sgRNA comprising the sequence of SEQ ID NO: 8 into the T cell for editing the; and/or
(iii) introducing a sgRNA comprising the sequence of SEQ ID NO: 15 into the T cell for editing the PD-1 genomic region from base 242800936 to 242800978 on chromosome 2, or the PD-1 genomic region from base 242795009 to 242795051 on chromosome 2.

In some embodiments, the invention provides a method for preparing a genetically modified T cell by a CRISPR/Cas9 genome editing technology, wherein:
(i) introducing a sgRNA comprising the sequence of SEQ ID NO: 2 into the T cell for editing the TRAC genomic region from base 23016448 to 23016490 on chromosome 14;
(ii) introducing a sgRNA comprising the base of SEQ ID NO: 8 into the T cell for editing the B2M genomic region from base 45003745 to 45003788 on chromosome 15; and/or
(iii) introducing a sgRNA comprising the sequence of SEQ ID NO: 14 into the T cell for editing the PD-1 genomic region from base 242800936 to 242800978 on chromosome 2, or the PD-1 genomic region from base 242795009 to 242795051 on chromosome 2.

In some embodiments, the invention provides a method for preparing a genetically modified T cell by a CRISPR/Cas9 genome editing technology, wherein:
(i) introducing a sgRNA comprising the sequence of SEQ ID NO: 3 into the T cell for editing the TRAC genomic region from base 23016448 to 23016490 on chromosome 14;
(ii) introducing a sgRNA comprising the sequence of SEQ ID NO: 7 into the T cell for editing the B2M genomic region from base 45003745 to 45003788 on chromosome 15; and/or
(iii) introducing a sgRNA comprising the sequence of SEQ ID NO: 15 into the T cell for editing the PD-1 genomic region from base 242800936 to 242800978 on chromosome 2, or the PD-1 genomic region from base 242795009 to 242795051 on chromosome 2.

In some embodiments, the TRAC-targeted sgRNA is introduced separately into the T cell. In some embodiments, the B2M-targeted sgRNA is introduced separately into the T cell. In some embodiments, the PD-1-targeted sgRNA is introduced separately into the T cell. In some embodiments, the TRAC-targeted sgRNA and the B2M-targeted sgRNA are simultaneously introduced into the T cell. In some embodiments, the TRAC-targeted sgRNA and the PD-1-targeted sgRNA are simultaneously introduced into the T cell. In some embodiments, the B2M-targeted sgRNA and the PD-1-targeted sgRNA are simultaneously introduced into the T cell. In some embodiments, the TRAC-targeted sgRNA, the B2M-targeted sgRNA, and the PD-1-targeted sgRNA are simultaneously introduced into the T cell.

In some embodiments, the sgRNA (including the TRAC-targeted sgRNA, the B2M-targeted sgRNA and/or the PD-1-targeted sgRNA) is a 2'-O-methyl sgRNA analog and/or an internucleotide 3'-thio sgRNA. In some embodiments, the sgRNA (including the TRAC-targeted sgRNA, the B2M-targeted sgRNA and/or the PD-1-targeted sgRNA) has 2'-O-methyl nucleotide analogs on the first one, two, and/or three base(s) of the 5' end and/or the last base of the 3' end.

In some embodiments, the sgRNA described above (including the TRAC-targeted sgRNA, the B2M-targeted sgRNA and/or the PD-1-targeted sgRNA) is introduced into the T cell by electroporation. In some embodiments, the above sgRNA (including the TRAC-targeted sgRNA, the B2M-targeted sgRNA and/or the PD-1-targeted sgRNA) and a Cas9-encoding nucleotide sequence (e.g., mRNA) are co-introduced into the T cell by electroporation. In some embodiments, the electroporation conditions comprise any one selected from the groups consisting of: 150-250V, 0.5-2ms; 150V, 2ms; 160V, 2ms; 170V, 2ms; 180V, 2ms; 190V, 1ms; 200V, 1ms ; 210V, 1ms; 220V, 1ms; 230V, 1ms; 240V, 1ms; 250V, 0.5ms.

In some embodiments, further comprising screening a T cell with low expression level of TRAC, B2M and/or PD-1 from genetically modified T cells. For example, the expression level of the TRAC, B2M or PD-1 in the genetically edited T cell is 1/10 of the expression level in a non-gene-edited T cell.

In some embodiments, the efficiency of a single gene knockout (TRAC, B2M, or PD-1) is 80% or more, such as 80%-100%, 85%-100%, 90%-100%, 95%-100% , 81% or more, 82% or more, 83% or more, 84% or more, 85% or more, 86% or more, 87% or more, 88% or more, 89% or more, 90% or more, 91% or more, 92% or more, 93 or more, 94% or more, 95% or more, 96% or more, 97% or more, 98% or more, or 99% or more; the efficiency of simultaneous knockout of double genes (such as TRAC and B2M) is 65% or more, for example, 65%-100%, 70%-100%, 75%-100%, 80%-100%, 85%-100%, 90%-100%, 95%-100%, 70% or more, 75% or more, 80% or more, 85% or more, 90% or more, or 95% or more; the efficiency of simultaneous knockout of the TRAC, B2M and PD-1 genes is 50% or more, for example, 55%-100%, 60%-100%, 65%-100%, 70%-100%, 75%-100%, 80%-100%, 85%-100%, 90%-100%, 95%-100%, 55% or more, 60% or more, 70% or more, 75% or more, 80% or more, 85% or more, 90% or more, or 95% or more. All numbers herein represent the number themselves and all the integers and decimals between the numbers. The knockout efficiency herein encompasses the knockout efficiency in the case of knockout of only one of, simultaneous knockout of two of, and simultaneous knockout of all three of the TRAC gene, the B2M gene, and the PD-1 gene.

In some embodiments, the T cell is derived from a healthy subject, a tumor or a viral infected patient (e.g., an HIV infected patient). In some embodiments, the T cell is a T cell differentiated from a stem cell or a precursor cell at different stages of differentiation.

In one aspect, the invention relates to a genetically modified T cell prepared by the above method.

In one aspect, the invention relates to a genetically modified T cell, wherein in the T cell:
(i) one or more loci in the TRAC genomic region from base 23016448 to 23016490 on chromosome 14 are disrupted by genome editing technology;
(ii) one or more loci in the B2M genomic region from base 45003745 to 45003788 on chromosome 15 are disrupted by genome editing technology; and/or
(iii) one or more loci in the PD-1 genomic region from base 242800936 to 242800978 on chromosome 2, or the PD-1 genomic region from base 242795009 to 242795051 on chromosome 2 are disrupted by genome editing technology. In some embodiments, the T cell is prepared by any of the methods described herein.

In one aspect, the invention relates to a genetically modified T cell, wherein in the T cell:
(i) the TRAC genomic region from base 23016448 to 23016490 on chromosome 14 has any one of the gene sequence changes listed in Tables D and E;
(ii) the B2M genomic region from base 45003745 to 45003788 on chromosome 15 has any one the gene sequence changes as listed Tables B and C; and/or
(iii) the PD-1 genomic region from base 242800936 to 242800978 on chromosome 2 has any one of the gene sequence changes listed in Table F, or the PD-1 genomic region from base 242795009 to 242795051 on chromosome 2 has any one of the gene sequence changes listed in Table G. In some embodiments, the T cell is prepared by any of the methods described herein.

In one aspect, the invention relates to the use of the above genetically modified T cell for the preparation of a T cell for adoptive cell therapy. In some embodiments, the T cell for adoptive cell therapy is a CAR-T cell or a TCR-T cell.

In one aspect, the invention relates to a method for preparing a CAR-T cell or a TCR-T cell, which comprises: introducing a chimeric antigen receptor (CAR) or the nucleotides encoding the chimeric antigen receptor, or an engineered T cell receptor (TCR) or the nucleotides encoding the T cell receptor into any one of the genetically modified T cells described above.

In one aspect, the invention relates to a method for preparing a CAR-T or TCR-T cell, which comprises:
(i) introducing a sgRNA comprising a sequence targeting the TRAC gene from base 23016448 to 23016490 on chromosome 14 into the T cell to disrupt the TRAC genomic region; and/or
(ii) introducing a sgRNA comprising a sequence targeting the B2M genomic region from base 45003745 to 45003788 on chromosome 15 into the T cell to disrupt the B2M genomic region; and/or
(iii) introducing a sgRNA comprising a sequence targeting the PD-1 genomic region from base 242800936 to 242800978 on chromosome 2, or the PD-1 genomic region from base 242795009 to 242795051 to the T cell to disrupt the B2M genomic region; and
(iv) introducing a chimeric antigen receptor (CAR) or the nucleotides encoding the chimeric antigen receptor, or an engineered T cell receptor (TCR) or the nucleotides encoding the T cell receptor into the T cell.
(iv) introducing a chimeric antigen receptor (CAR) or the nucleotides encoding the chimeric antigen receptor, or an engineered T cell receptor (TCR) or the nucleotides encoding the T cell receptor into the T cell. In some embodiments, the method further comprises introducing Cas9 or the nucleotides encoding Cas 9 into the T cell.

In some embodiments, the method for preparing a CAR-T or TCR-T cell comprises:
(i) introducing a sgRNA comprising any one selected from SEQ ID NOs: 2-5 into the T cell for editing the TRAC genomic region from base 23016448 to 23016490 on chromosome 14;
(ii) introducing a sgRNA comprising any one selected from SEQ ID NOs: 6-13 into the T cell for editing the B2M genomic region from base 45003745 to 45003788 on chromosome 15; and/or
(iii) introducing a sgRNA comprising any one selected from SEQ ID NOs: 14-22 into the T cell for editing the PD-1 genomic region from base 242800936 to 242800978 on chromosome 2, or the PD-1 genomic region from base 242795009 to 242795051 on chromosome 2; and
(iv) introducing a chimeric antigen receptor (CAR) or the nucleotides encoding the chimeric antigen receptor, or an engineered T cell receptor (TCR) or the nucleotides encoding the T cell receptor into the T cell. In some embodiments, the method further comprises introducing Cas9 or the nucleotides encoding Cas 9 into the T cell.

In some embodiments, the method for preparing a CAR-T or TCR-T cell comprises:
(i) introducing a sgRNA comprising a sequence selected from any one of SEQ ID NO: 2 or SEQ ID NO: 3 into the T cell for editing the TRAC genomic region from base 23016448 to 23016490 on chromosome 14;
(ii) introducing a sgRNA comprising a sequence selected from any one of SEQ ID NO: 7 or SEQ ID NO: 8 into the T cell for editing the B2M genomic region from base 45003745 to 45003788 on chromosome 15; and/or
(iii) introducing a sgRNA comprising a sequence selected from any one of SEQ ID NO: 14 or SEQ ID NO: 15 into the T cell for editing the PD-1 genomic region from base 242800936 to 242800978 on chromosome 2, or the PD-1 genomic region from base 242795009 to 242795051 on chromosome 2; and
(iv) introducing a chimeric antigen receptor (CAR) or the nucleotides encoding the chimeric antigen receptor, or an engineered T cell receptor (TCR) or the nucleotides encoding the T cell receptor into the T cell. In some embodiments, the method further comprises introducing Cas9 or the nucleotides encoding Cas 9 into the T cell.

In some embodiments, the method for preparing a CAR-T or TCR-T cell comprises:
(i) introducing a sgRNA comprising the sequence of SEQ ID NO: 2 into the T cell for editing the TRAC genomic region from base 23016448 to 23016490 on chromosome 14;
(ii) introducing a sgRNA comprising the sequence of SEQ ID NO: 8 into the T cell for editing the B2M genomic region from base 45003745 to 45003788 on chromosome 15; and/or
(iii) introducing a sgRNA comprising the sequence of SEQ ID NO: 15 into the T cell for editing the PD-1 genomic region from base 242800936 to 242800978 on chromosome 2, or the PD-1 genomic region from base 242795009 to 242795051 on chromosome 2; and
(iv) introducing a chimeric antigen receptor (CAR) or the nucleotides encoding the chimeric antigen receptor, or an engineered T cell receptor (TCR) or the nucleotides encoding the T cell receptor into the T cell. In some embodiments, the method further comprises introducing Cas9 or the nucleotides encoding Cas 9 into the T cell.

In some embodiments, the method for preparing a CAR-T or TCR-T cell comprises:
(i) introducing a sgRNA comprising the sequence of SEQ ID NO: 2 into the T cell for editing the TRAC genomic region from base 23016448 to 23016490 on chromosome 14;
(ii) introducing a sgRNA comprising the sequence of SEQ ID NO: 8 into the T cell for editing the B2M genomic region from base 45003745 to 45003788 on chromosome 15; and/or
(iii) introducing a sgRNA comprising the sequence of SEQ ID NO: 14 into the T cell for editing the PD-1 genomic region from base 242800936 to 242800978 on chromosome 2, or the PD-1 genomic region from base 242795009 to 242795051 on chromosome 2; and
(iv) introducing a chimeric antigen receptor (CAR) or the nucleotides encoding the chimeric antigen receptor, or an engineered T cell receptor (TCR) or the nucleotides encoding the T cell receptor into the T cell. In some embodiments, the method further comprises introducing Cas9 or the nucleotides encoding Cas 9 into the T cell.

In some embodiments, the method for preparing a CAR-T or TCR-T cell comprises:
(i) introducing a sgRNA comprising the sequence of SEQ ID NO: 3 into the T cell for editing the TRAC genomic region from base 23016448 to 23016490 on chromosome 14;
(ii) introducing a sgRNA comprising the sequence of SEQ ID NO: 7 into the T cell for editing the B2M genomic region from base 45003745 to 45003788 on chromosome 15; and/or
(iii) introducing a sgRNA comprising the sequence of SEQ ID NO: 15 into the T cell for editing the PD-1 genomic region from base 242800936 to 242800978 on chromosome 2, or the PD-1 genomic region from base 242795009 to 242795051 on chromosome 2; and
(iv) introducing a chimeric antigen receptor (CAR) or the nucleotides encoding the chimeric antigen receptor, or an engineered T cell receptor (TCR) or the nucleotides encoding the T cell receptor into the T cell. In some embodiments, the method further comprises introducing Cas9 or the nucleotides encoding Cas 9 into the T cell.

In some embodiments, the TRAC-targeted sgRNA and the B2M-targeted sgRNA are simultaneously introduced into the T cell. In some embodiments, the TRAC-targeted sgRNA, the B2M-targeted sgRNA, and the PD-1-targeted sgRNA are simultaneously introduced into the T cell.

In some embodiments, the sgRNA (including the TRAC-targeted sgRNA, the B2M-targeted sgRNA and/or the PD-1-targeted sgRNA) is a 2'-O-methyl sgRNA analog and/or nucleotide 3'-thio sgRNA. In some embodiments, the sgRNA (including the TRAC-targeted sgRNA, the B2M-targeted sgRNA and/or the PD-1-targeted sgRNA) has 2'-O-methyl nucleotide analogs on the first one, two, and/or three base(s) of the 5' end and/or the last base of the 3' end.

In some embodiments, the sgRNA described above (including the TRAC-targeted sgRNA, the B2M-targeted sgRNA and/or the PD-1-targeted sgRNA) is introduced into the T cell by electroporation. In some embodiments, the above sgRNAs (including the TRAC-targeted sgRNA, the B2M-targeted sgRNA and/or the PD-1-targeted sgRNA) and a Cas9-encoding nucleotide sequence (e.g., mRNA) are co-introduced into the T cell by electroporation. In some embodiments, the electroporation conditions comprise any one selected from the groups consisting of: 150-250V, 0.5-2ms; 180-250V, 0.5-2ms; 150V, 2ms; 160V, 2ms; 170V, 2ms; 180V, 2ms ;190V, 1ms; 200V, 1ms; 210V, 1ms; 220V, 1ms; 230V, 1ms; 240V, 1ms; 250V, 0.5ms.

In some embodiments, the methods comprise simultaneously introducing the TRAC-targeted sgRNA, the B2M-targeted sgRNA and/or the PD-1-targeted sgRNA, and the CAR or its encoding nucleotide sequence, or an engineered T cell receptor (TCR) or the nucleotides encoding the T cell receptor into the T cell.

In some embodiments, introducing the CAR or its encoding nucleotide sequence, or an engineered T cell receptor (TCR) or the nucleotides encoding the T cell receptor into the T cell, prior to introducing the TRAC-targeted sgRNA, the B2M-targeted sgRNA, and/or the PD-1-targeted sgRNA into it; or introducing the CAR or its encoding nucleotide sequence, or an engineered T cell receptor (TCR) or the nucleotides encoding the T cell receptor into the T cell, after introducing the TRAC-targeted sgRNA, the B2M-targeted sgRNA, and/or the PD-1-targeted sgRNA into it.

In one aspect, the invention relates to a CAR-T cell or a TCR-T cell prepared by the above method.

In one aspect, the invention relates to a CAR-T cell, which comprises the above genetically modified T cell expressing a chimeric antigen receptor (CAR).

In one aspect, the invention relates to a CAR-T cell, wherein in the CAR-T cell:
(i) the TRAC genomic region from base 23016448 to 23016490 on chromosome 14 has any one of the gene sequence changes listed in Tables D and E;
(ii) the B2M genomic region from base 45003745 to 45003788 on chromosome 15 has any one of the gene sequence changes listed in Tables B and C; and/or
(iii) the PD-1 genomic region from base 242800936 to 242800978 on chromosome 2 has any one of the gene sequence changes listed in Table F, or the PD-1 genomic region from base 242795009 to 242795051 on chromosome 2 has any one of the gene sequence changes listed in Table G.

In one aspect, the invention relates to a TCR-T cell, which comprises the above genetically modified T cell expressing an engineered TCR.

In one aspect, the invention relates to a TCR-T cell, wherein in the TCR-T cell:
(i) the TRAC genomic region from base 23016448 to 23016490 on chromosome 14 has any one of the gene sequence changes listed in Tables D and E;
(ii) the B2M genomic region from base 45003745 to 45003788 on chromosome 15 has any one of the gene sequence changes listed in Tables B and C; and/or
(iii) the PD-1 genomic region from base 242800936 to 242800978 on chromosome 2 has any one of the gene sequence changes listed in Table F, or the PD-1 genomic region from base 242795009 to 242795051 on chromosome 2 has any one of the gene sequence changes listed in Table G.

The location information of the TRAC genomic region, the B2M genomic region, and the PD-1 genomic region mentioned above in the present invention is determined according to the sequence location information of the wild type genes described in the reference database: GRCh37 (hg19). Those skilled in the art know how to obtain corresponding location information of the above-described genomic regions with reference to other databases.

In some particular embodiments, the wild type nucleotide sequence of the TRAC genomic region from base 23016448 to 23016490 on chromosome 14 is represented by SEQ ID NO: 23 (tatccagaaccctgaccctgccgtgtaccagctgagagactct). In some particular embodiments, the wild type nucleotide sequence of the B2M genomic region from base 45003745 to 45003788 on chromosome 15 is represented by SEQ ID NO: 24 (atgtctcgctccgtggccttagctgtgctcgcgctactctctct). In some particular embodiments, the wild nucleotide sequence of the PD-1 genomic region from base 242800936 to 242800978 on chromosome 2 is represented by SEQ ID NO: 25 (agcccagttgtagcaccgcccagacgactggccagggcgcctg). In some particular embodiments, the wild nucleotide sequence of the PD-1 genomic region from base 242795009 to 242795051 on chromosome 2 is represented by SEQ ID NO: 26 (cagtttagcacgaagctctccgatgtgttggagaagctgcagg).

In one aspect, the invention also relates to a composition (e.g., a pharmaceutical combination), a kit, and a medical product comprising the above-described genetically modified T cell, CAR-T cell, or TCR-T cell.

In one aspect, the invention relates to a method for treating a disease in a subject, which comprises administering to the subject an effective amount of the above CAR-T or TCR-T cells. In some embodiments, the disease is a tumor. In some embodiments, the tumor is a hematological tumor. In some embodiments, the tumor is lymphoma or leukemia. In some embodiments, the CAR targets a tumor specific antigen (TSA) and/or a tumor associated antigen (TAA), such as an antigen (e.g., CD19) as shown in Table A herein.

In one aspect, the invention relates to a sgRNA comprising any one of SEQ ID NOs: 2-22 or a vector thereof. In some embodiments, the sgRNA is chemically modified, such as a 2'-O-methyl sgRNA analog and/or an internucleotide 3'-thio sgRNA. In some embodiments, the sgRNA has 2'-O-methyl nucleotide analogs on the first one, two, and/or three base(s) of the 5' end and/or the last base of the 3' end.

### BRIEF DESCRIPTION OF THE DRAWINGS

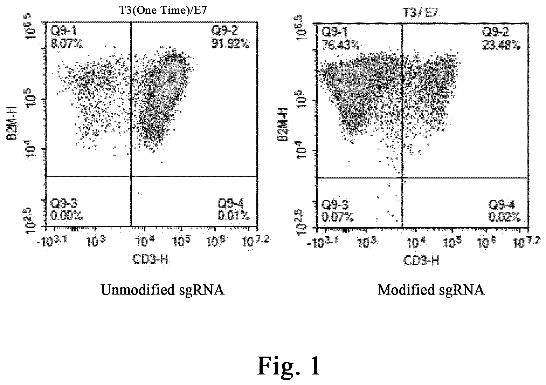

Figure 1 shows comparison of the knockout efficiency of corresponding loci after one time of electroporation by using sgRNA with or without chemical modification.

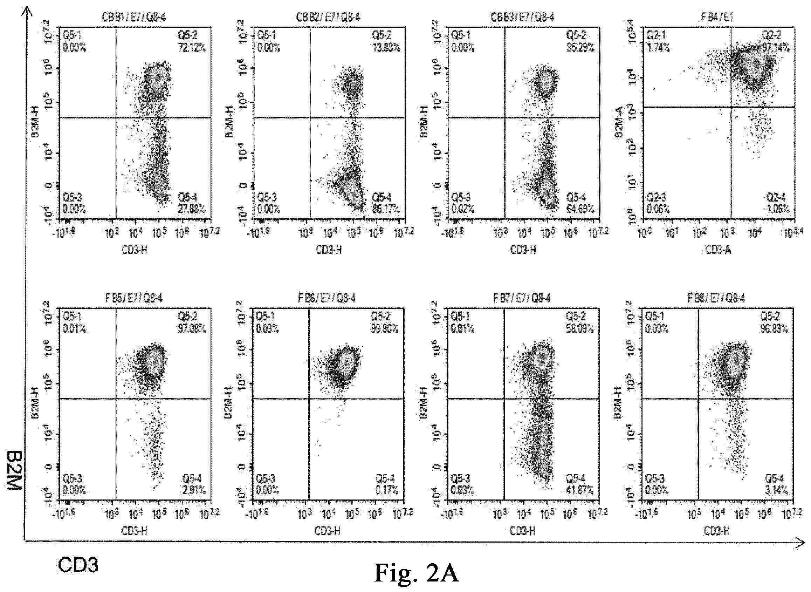

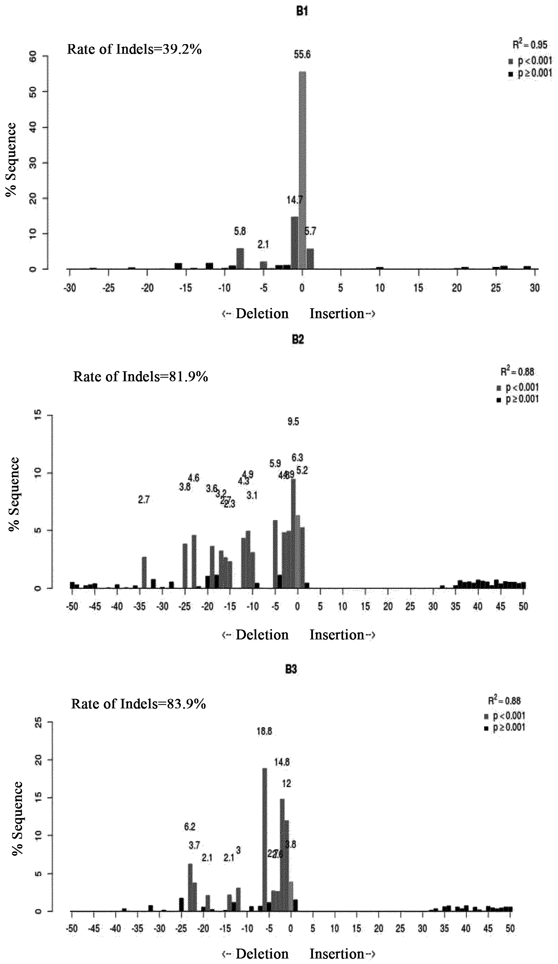

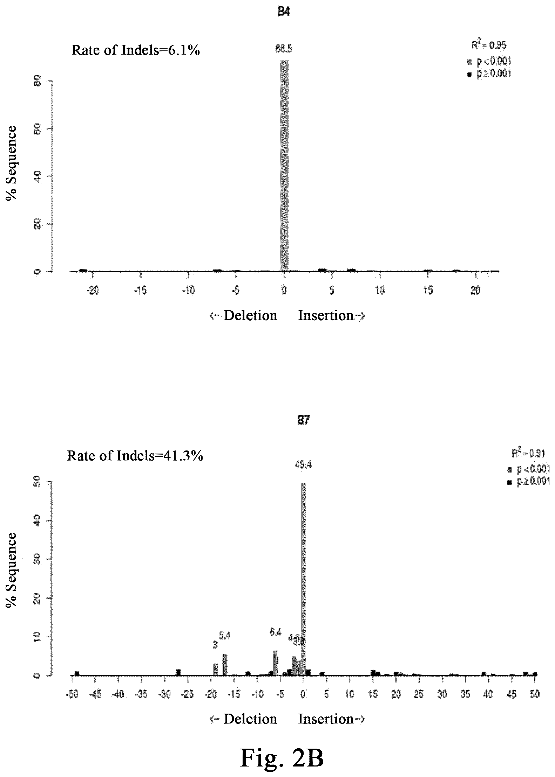

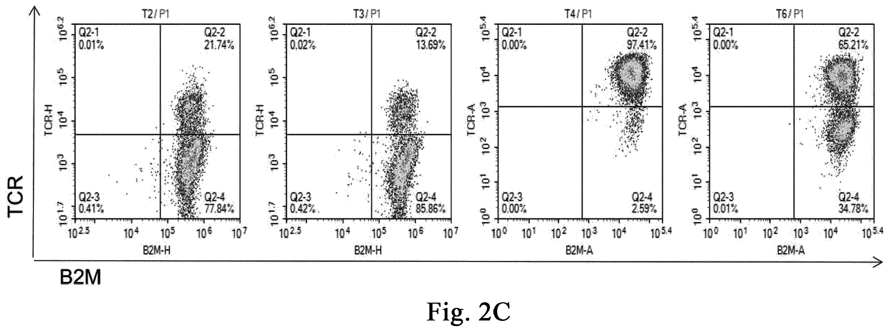

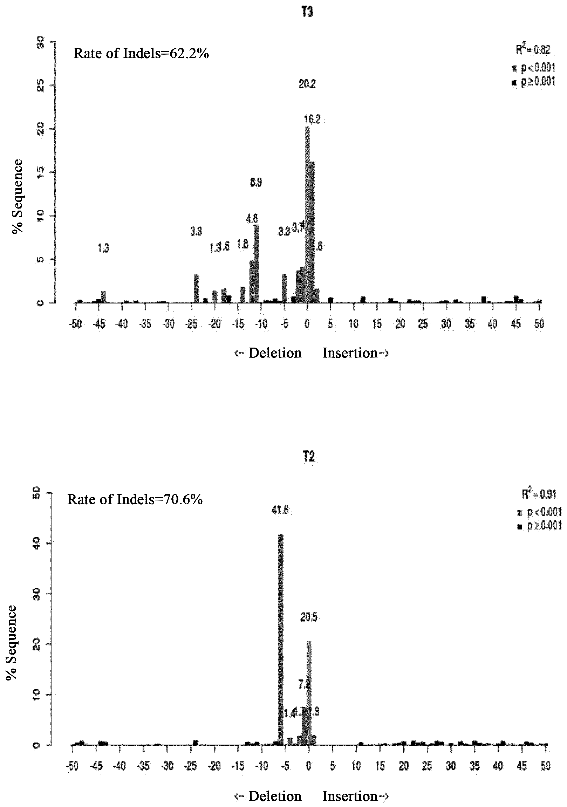

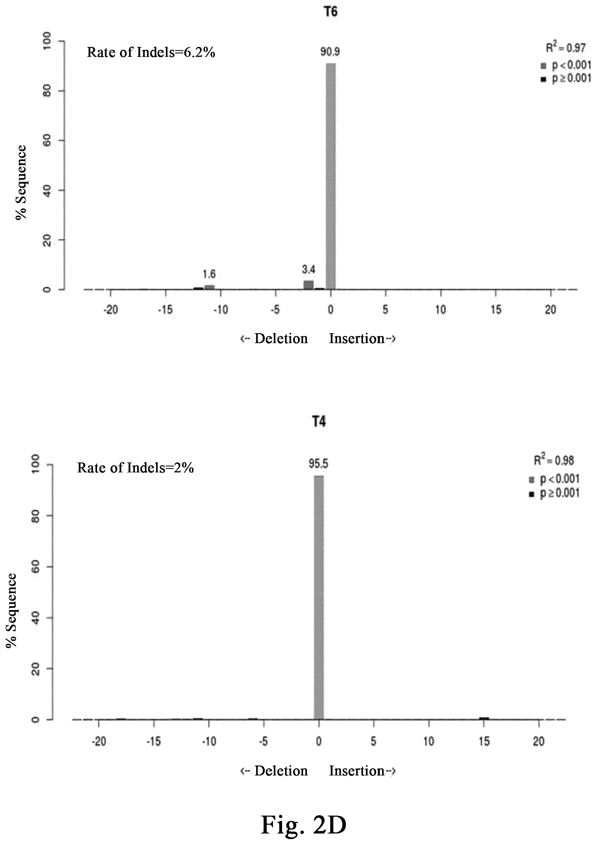

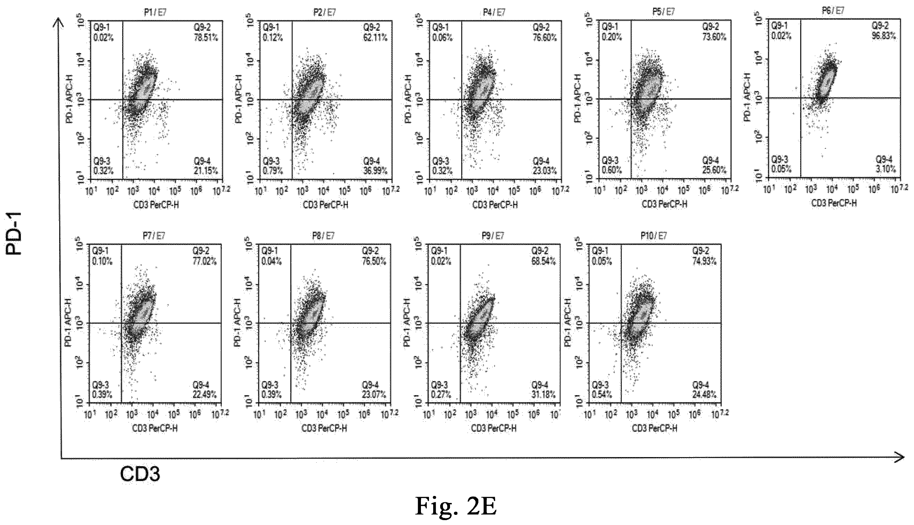

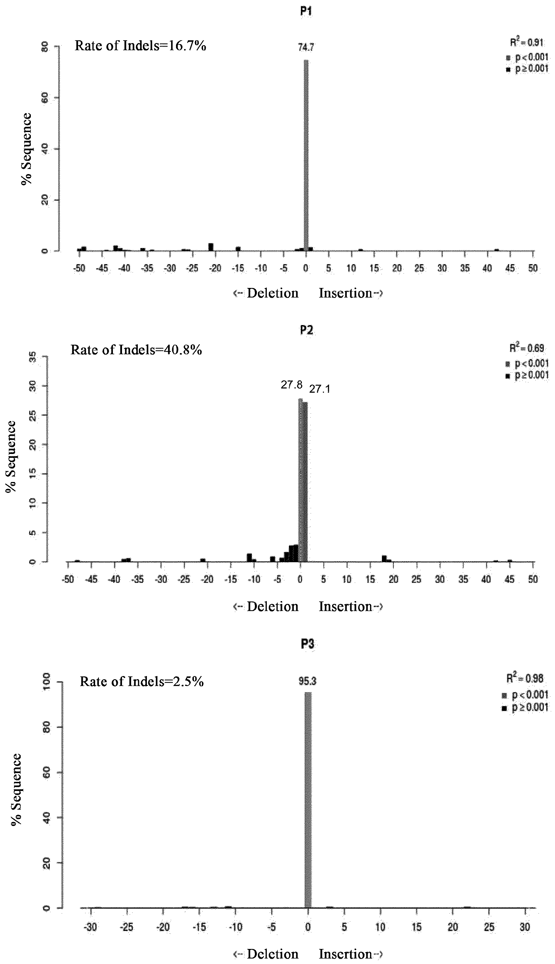

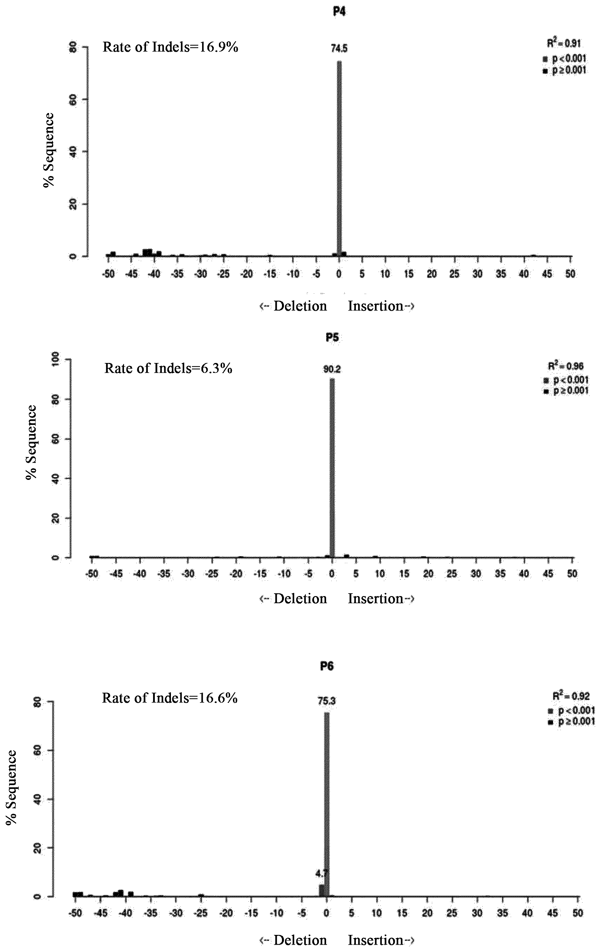

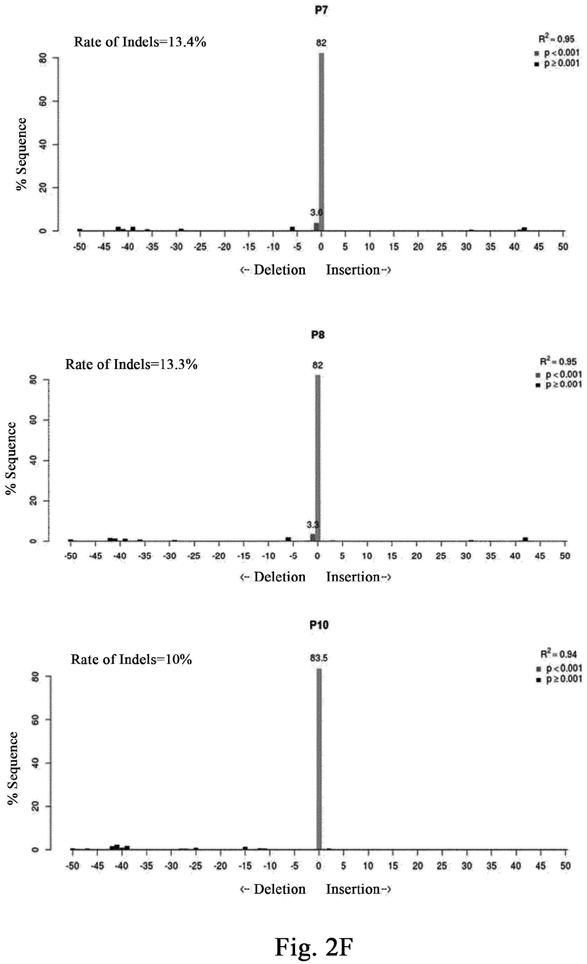

Figures 2A-2F. Figure 2A shows the analysis on the knockout efficiency of each sgRNA for B2M gene after the knockout of B2M in HLA by using different modified sgRNAs in conjunction with the CRISPR/Cas9 gene knockout tool. Figure 2B shows the INDEL analysis by the INDEL analysis software on the T cell genome after the knockout of the B2M gene by different modified sgRNAs and the rate of INDEL obtained in the T cell. Figure 2C shows the analysis on the knockout efficiency of each sgRNA for TRAC gene after the knockout of TRAC in TCR by using different modified sgRNAs in conjunction with the CRISPR/Cas9 gene knockout tool. Figure 2D shows the INDEL analysis by the INDEL analysis software on the T cell genome after the knockout of the TRAC gene by different modified sgRNAs, and the rate of INDEL obtained in the T cell. Figure 2E shows the analysis on the knockout efficiency of each sgRNA for PD-1 gene after the knockout of PD-1 by using different modified sgRNAs in conjunction with the CRISPR/Cas9 gene knockout tool. Figure 2F shows the INDEL analysis by the INDEL analysis software on the T cell genome after the knockout of the PD-1 gene by different modified sgRNAs and the rate of INDEL obtained in the T cell.

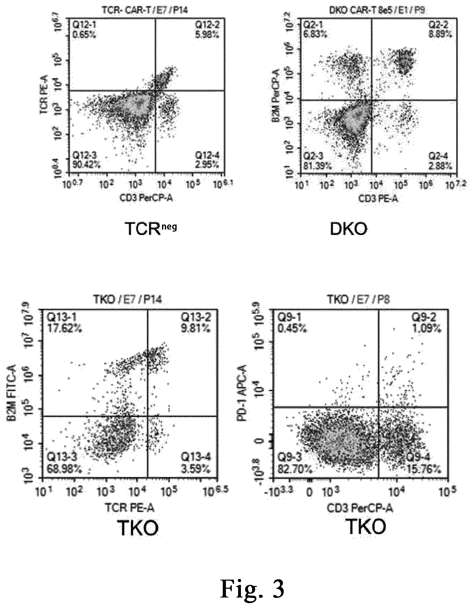

Figure 2A shows the knockout of B2M in the T cell under the same electroporation conditions by using *in vitro* transcription (IVT) and the modified and optimized sgRNA in conjunction with the CRISPR/Cas9 gene knockout tool. The knockout efficiency of each sgRNA for B2M gene with analysis after the knockout are respectively: B1: 27.88%, B2: 86.17%, B3: 64.69%, B4: 1.06%, B5: 2.91%, B6: 0.17%, B7: 41.87%, and B8: 3.14%. As shown in the figure, a sgRNA with better knockout efficiency can be selected. It is possible to provide a high efficient double/triple gene knockout (shown in Figure 3) by a selected optimized 2'-O-methyl sgRNA analog and/or an internucleotide 3'-thio sgRNA. Figure 2B shows the INDEL analysis by the INDEL analysis software on the T cell genome after the knockout of the B2M gene by different sgRNAs, thereby obtaining the rate of INDEL in the T cell. Figure 2C shows the knockout of TRAC in the T cell under the same electroporation conditions by using *in vitro* transcription (IVT) and the modified and optimized sgRNA in conjunction with the CRISPR/Cas9 gene knockout tool. The knockout efficiency of each sgRNA for TRAC gene with analysis after the knockout are respectively: T2: 77.84%, T3: 85.86%, T4: 2.59%, and T6: 34.78%. As shown in the figure, a sgRNA with better knockout efficiency can be selected. It is possible to provide a highly efficient single/double/triple gene knockout (shown in Figure 3) by optimized modification of a selected sgRNA as mentioned above. Figure 2D shows the INDEL analysis by the INDEL analysis software on the T cell genome after the knockout of the TRAC gene by different sgRNAs, thereby obtaining the rate of INDEL in the T cell. Figure 2E shows the knockout of PD-1 in the T cell under the same electroporation conditions by using *in vitro* transcription (IVT), the modified and optimized sgRNA in conjunction with the CRISPR/Cas9 gene knockout tool, and the knockout efficiency of each sgRNA for PD-1 gene with analysis after the knockout are respectively: P1: 21.15%, P2: 36.99%, P4: 23.03%, P5: 25.6%, P6: 3.1%, P7: 22.49%, P8: 23.07%, P9 : 31.18%, and P10: 24.48%. As shown in the figure, a sgRNA with better knockout efficiency can be selected. It is possible to provide a high efficient triple gene knockout (shown in Figure 3) by optimized modification of a selected sgRNA as mentioned above. Figure 2F shows the INDEL analysis by the INDEL analysis software on the T cell genome after the knockout of the PD-1 gene by different sgRNAs, thereby obtaining the rate of INDEL in the T cell.
Figure 3 shows the analysis on the results of the knockout of TRAC, TRAC/B2M (Double Knock-Out, DKO), and TRAC/B2M/PD-1 (Triple Knock-Out, TKO) for the T cell by using optimized sgRNA and CRISPR/Cas9 genome editing technology. The sgRNAs are chemically modified, and then the chemically modified sgRNAs and Cas9 are delivered to primary T cells by further optimized electroporation conditions for knockout of the related gene. As shown in the figure, the knockout efficiency of the single gene (i.e., TRAC) is increased to 90.42%; the knockout efficiency of the two genes (i.e., TRAC and B2M) is increased to 81.39%; and the knockout efficiency of the three genes (i.e., TRAC, B2M and PD-1) is increased to 67.91% ((82.70%+15.76%)^{∗}68.98%).

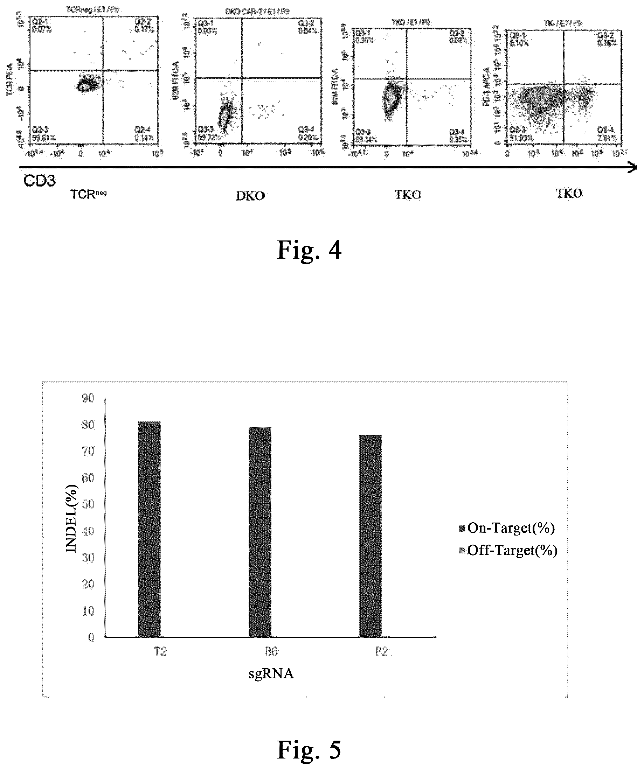

Figure 4 shows phenotypic analysis of T cells with knockout of TRAC, TRAC/B2M (DKO), and TRAC/B2M/PD-1 (TKO) after screening and purification. By optimizing the purification conditions in the later stage, i.e., increasing the number of purification times (4-5 times) and the corresponding amount of antibody (3 mg/mL), it is shown in the figure that after optimization the purity of the double-gene knockout (i.e., TRAC and B2M double-gene knockout) increases to 99.72%; and the purity of the three-gene knockout (i.e., TRAC, B2M and PD-1 gene knockout) increases to 98.62%.
Figure 5 shows the results of off-target detection of TRAC-sgRNA3 (T2), B2M-sgRNA2 (B3), and PD-1-sgRNA2 (P2). The figure shows the rate of INDEL in the T cell edited by T2, B3, P2 sgRNA in conjunction with CRISPR/Cas9, and the results are obtained by using INDEL analysis software after deep sequencing.

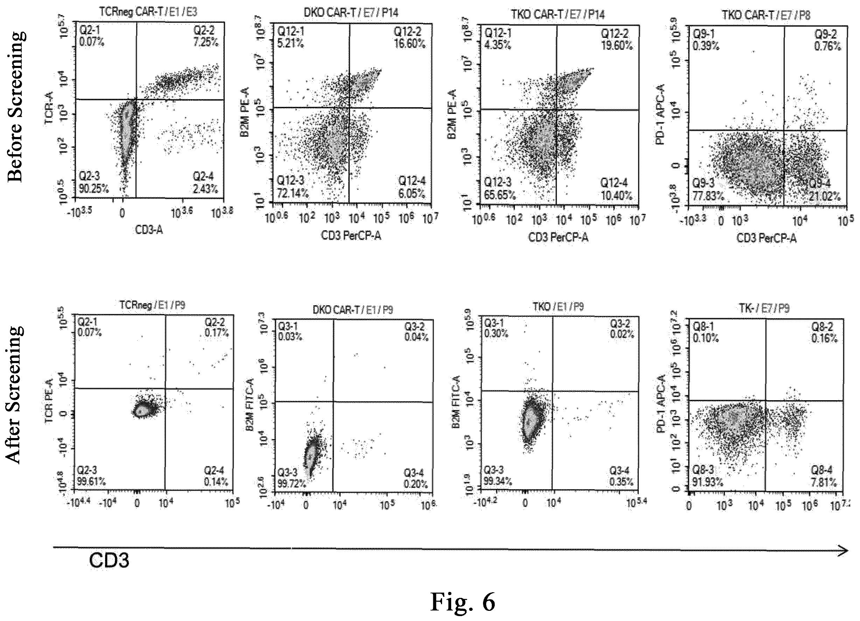

Figure 6 shows phenotypic analysis of T cells with knockout of TCR and/or B2M and/or PD-1, and phenotypic analysis of T cells with knockout of TCR and/or B2M and/or PD-1 after screening and purification, by using optimized sgRNA and CRISPR/Cas9 genome editing technology for knockout. The horizontal axis is CD3, and the vertical axis is TCR, B2M or PD-1.

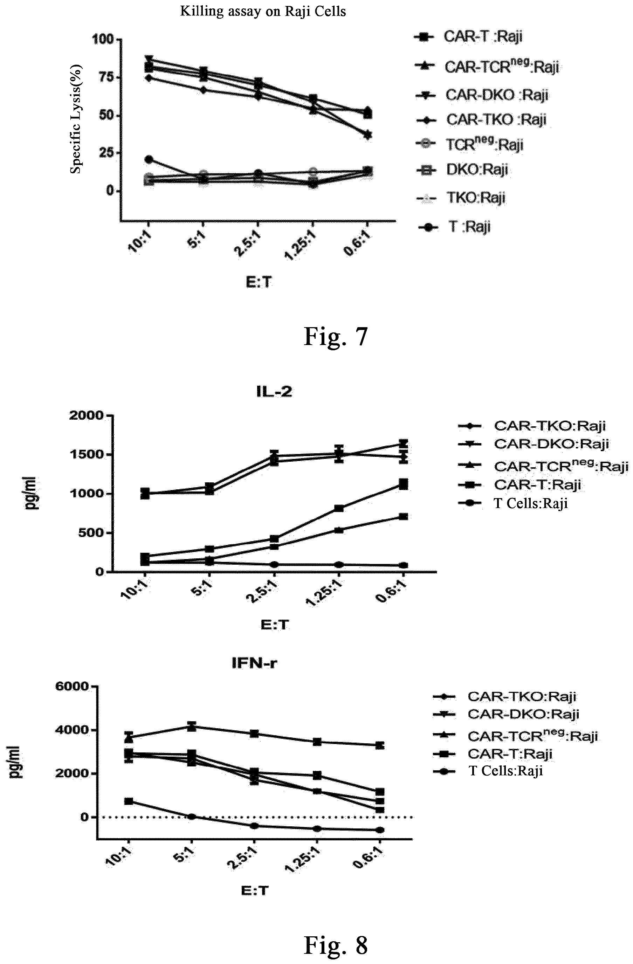

Figure 7 shows the validation and comparison of the killing functions of the T cell, CAR-T, TCRneg CAR-T, DKO CAR-T and TKO CAR-T cell. In this experiment, Raji and K562 are used as target cells, and T, CAR-T, DKO CAR-T and TKO CAR-T are effector cells; and the *in vitro* killing assays are performed respectively with the effector-to-target ratios of 10:1, 5:1, 2.5:1, 1.25: 1, 0.625: 1.
Figure 8 shows the cytokine release of T cell, CAR-T, TCRneg CAR-T, DKO CAR-T, and TKO CAR-T. In this experiment, Raji and K562 are used as target cells, and T, CAR-T, DKO CAR-T, and TKO CAR-T are effector cells; and after co-culturing *in vitro* respectively with the effector-to-target ratio of 10:1, 5:1, 2.5:1, 1.25: 1, 0.625:1, the supernatants are taken for detection of IL-2 and IFN-γ.

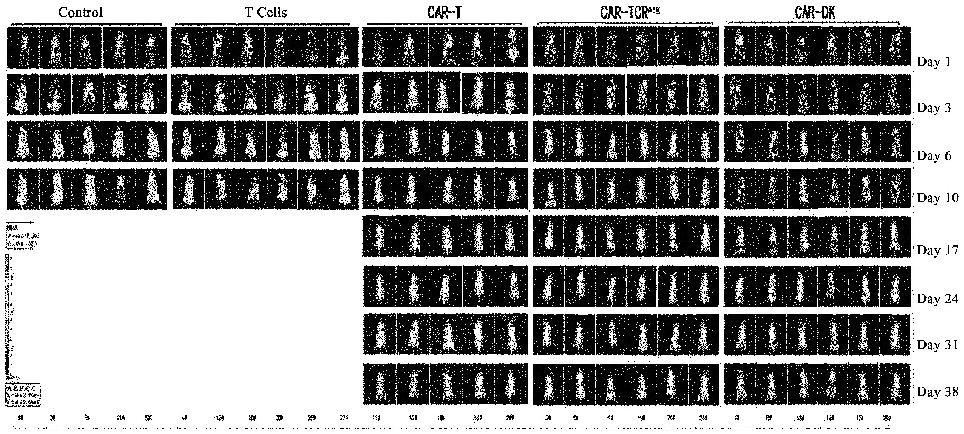

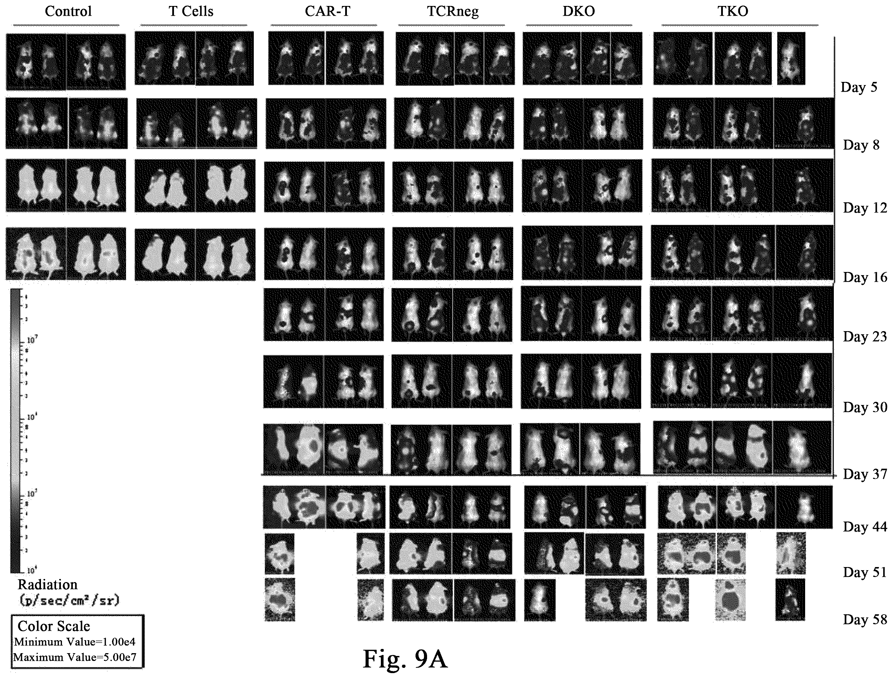

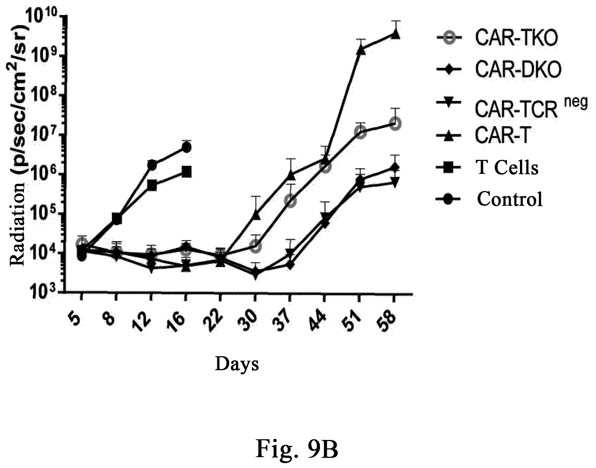

Figures 9A-9B show the comparison of the tumor suppression and killing efficacy in NPG mice after injection of saline, CAR-T, TCR^{neg} CAR-T, DKO CAR-T (TCR^{neg}\B2M^{neg}-CAR-T) or TKO CAR-T (TCR^{neg}\B2M^{neg}\PD-1^{neg}-CAR-T) cells. Figure 9A shows that NSC mice are randomly divided into 4 groups after intravenous injection of 5×10⁵ tumor cells per mouse, i.e., saline group, T cell group, TCR/CD3^{neg} CD19-CAR-T cell group, DKO CD19-CAR-T cell group, and the TKO CD19-CAR-T cell group, then 5×10⁶ corresponding cells are respectively injected into the four groups of mice through the tail vein, and the physiological saline group is used as a control group. The tumor burden in mice is analyzed using a picture obtained from a PerkinElmer imager. Figure 9B shows the tumor burden in mice by a picture obtained by a PerkinElmer imager, the horizontal axis is the number of days of mouse rearing after the cell injection, and the vertical axis is the radiation signal radiated from the unit body surface area per second.

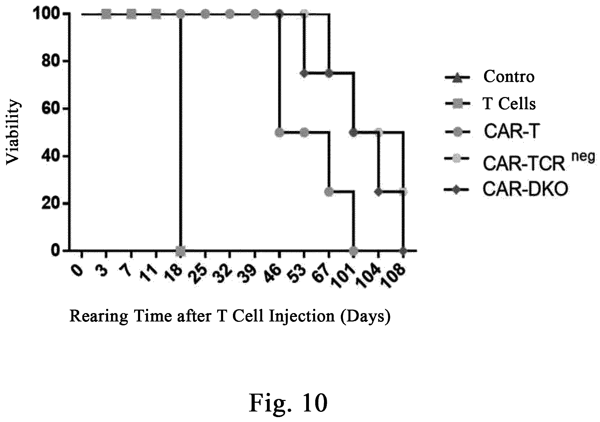

Figure 10 shows comparison of the mouse viability of the five mice groups: saline, CAR-T, TCR^{neg} CAR-T, DKO CAR-T (TCR^{neg}\B2M^{neg}-CAR-T), and TKO CAR-T (TCR^{neg}\B2M^{neg}\PD-1^{neg}-CAR-T). The horizontal axis is the number of days of mouse rearing after injection of the effector cells, and the vertical axis is the mouse viability.

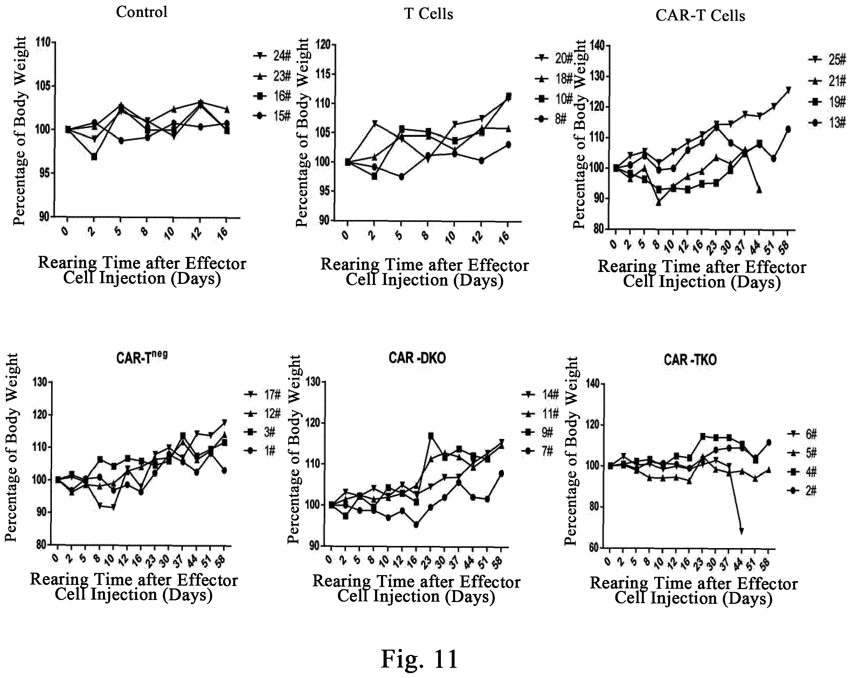

Figure 11 shows comparison of the results of body weight change in mice after injection of saline, CAR-T, TCR^{neg} CAR-T, DKO CAR-T (TCR^{neg}\B2M^{neg}-CAR-T), and TKO CAR-T (TCR^{neg}\B2M^{neg}\PD-1^{neg}-CAR-T) cells. Body weights of the NPG mice are monitored, the horizontal axis is the number of days of mouse rearing after injection, and the vertical axis is the percent body weight of mice (n=4).

### DETAILED DESCRIPTION OF THE INVENTION

### Definitions

As used herein, "CRISPR/Cas" is a genome editing technology including, but not limited to, a variety of naturally occurring or manually designed CRISPR/Cas systems, such as the CRISPR/Cas9 system. The naturally occurring CRISPR/Cas system is an adaptive immune defense formed during the long-term evolution of bacteria and archaea and are used to fight against the invading viruses and foreign DNA. For example, the mechanism of CRISPR/Cas9 is that crRNA (CRISPR-derived RNA) binds to tracrRNA (trans-activating crRNA) through base pairing to form a tracrRNA/crRNA complex, which directs the nuclease Cas9 protein to cleave double-stranded DNA at the target site of a sequence in pair with the crRNA. By artificially designing, tracrRNA and crRNA are engineered to bea sgRNA (single guide RNA) which is able to guide Cas9 to perform the site-directed cleavage of DNA. As an RNA-guided dsDNA-binding protein, Cas9 effector nucleases are capable of co-localizing RNA, DNA, and proteins, such that it has enormous potential for transformation. Type I, II and III Cas proteins can be used in CRISPR/Cas system.. In some embodiments of the invention, the Cas9 is used in the method herein. Other suitable CRISPR/Cas systems include, but are not limited to the systems and methods described in WO2013176772, WO2014065596, WO2014018423, and US8,697,359.

In the present invention, "sgRNA (single guide RNA)", "gRNA (guide RNA)" and "synthetic guide RNA" are used interchangeably. The sgRNA of the invention comprises a guide sequence that targets a target sequence.

"T cell receptor (TCR)" is a characteristic marker on the surface of all T cells, and it binds to CD3 to form a TCR-CD3 complex. TCR consists of two peptide chains: α and β. Each peptide chain is further divided into variable region (V region), constant region (C region), transmembrane region, and cytoplasmic region. The TCR molecule belongs to immunoglobulin superfamily, and its antigenic specificity exists in the V region; the V region (Vα, Vβ) has three hypervariable regions (i.e., CDR1, CDR2, and CDR3), among which the variation of CDR3 is the greatest and directly determines the antigen-binding specificity of the TCR. When TCR recognizes the MHC-antigen peptide complex, CDR1 and CDR2 recognize and bind to the side walls of the antigen binding groove of MHC molecule, and CDR3 binds directly to the antigen peptide. TCR is divided into two categories: TCR1 and TCR2. TCR1 consists of two chains: γ and δ, and TCR2 consists of two chains: α and β. In peripheral blood, 90%-95% of T cells express TCR2; and each T cell expresses only TCR2 or onlyTCR1.

"β2 microglobulin (B2M)", is the β-chain (light chain) part of a human leukocyte antigen (HLA) on cell surface. It is a single-chain polypeptide with a molecular mass of 11,800 and consisting of 99 amino acids.

"Programmed Death Receptor 1 (PD-1)" is a membrane protein with 268 amino acid residues. It is originally cloned from the apoptotic mouse T cell hybridoma 2B4.11. The combination of PD-1 and PD-L1 initiates programmed cell death of T cells, allowing tumor cells to achieve immune escape. Therefore, PD-1 is an important immunosuppressive molecule.

As used herein, "Indel" is a short form of insertion/deletion, i.e., an insertion and/or deletion mutation.

"Graft versus host disease (GVHD)" occurs when there's immunogenetic difference between the receptor and donor. For example, on the one hand, when donor cells, such as immunocompetent T lymphocytes from a donor, enter the body of a patient as a recipient and proliferate to a certain amount, the normal cells or tissues of the patient are mistaken for targets and are attacked. It results in GVHD.. On the other hand, as allogeneic cells, they may also be cleared by the normal immune system in the recipient that results in a "host versus graft reaction (HVGR)".

HVGR and GVHD-related genes contain TCR and HLA-related genes. T lymphocytes with simultaneous knockout of these genes do not cause graft-versus-host disease (GVHD) when they are infused to an allogeneic patient, so they can be called "universal T cells". For example, a single TRAC gene is the gene that encodes TCR α chain, which combines two TRBC genes which encodes TCRβ to form a complete functional TCR complex. Knockout of TRAC results in inactivation of TCR. While B2M is an MHC I-related gene. T lymphocytes with simultaneous knockout of the two genes do not cause graft-versus-host disease (GVHD) when they are infused to an allogeneic patient.

"CAR-T" is the abbreviation of "chimeric antigen receptor T cell", wherein the chimeric antigen receptor (CAR) is a core component of CAR-T, conferring T cell the ability of HLA-independent recognition of antigen of the target cells (e.g., tumor cells), thereby allowing the CAR-modified T cells to recognize a broader range of targets than the natural T cell surface receptor TCR does. In some embodiments, the design of a tumor-targeting CAR comprises a tumor-associated antigen (TAA) binding region (e.g., an scFV segment typically derived from a monoclonal antibody's antigen-binding region), an extracellular hinge region, a transmembrane region, and an intracellular signal region. The selection of the target antigen is a key determinant for the specificity and effectiveness of the CAR and the safety of the genetically modified T cells themselves.

"Universal CAR-T cells" refer to CAR-T cells capable of targeting specific target cell (e.g., tumor cell) associated markers and the functions of TCR and MHC on which are inactivate, thereby the immune rejection caused by allogeneic cell therapy is reduced.

CAR-T treatment with autologous cells requires the preparation of T lymphocytes isolated from the patients' own blood. On one hand, since each patient's own condition and the status of T lymphocytes are different, and many factors affecting the production process of CAR-T are not standardized, the production safety is affected. On the other hand, as some patients' autologous T lymphocytes have insufficient activity and quantity after chemotherapy, or the activity and proliferation ability of their T lymphocytes are limited by the affection of tumor environment, it is often difficult to prepare CAR-T from such cells, and the safety and effectiveness of treatment are affected. In addition, if there is an emergent situation during the preparation of CAR-T cells such that the prepared cells cannot be infused back to the patient in time, the efficiency of the therapy will be affected. Due to the affection of the state of autologous T lymphocytes, Some tumor patients cannot even accept autologous CAR-T adoptive cell therapy. Accordingly, universal CAR-T or universal T lymphocytes suitable for allogeneic treatment have a great advantage in the above circumstances.

TCR-T (T cell receptor (TCR) chimeric-T cell) refers to a T cell expressing an engineered TCR receptor (or called as artificial T cell receptor). The engineered T cell receptor or artificial T cell receptor is genetically modified to have a structure targeting the antigen of interest, and also retain the domains and/or accessory molecules in the TCR signaling pathway. In some embodiments, TCR-T retains all the accessory molecules in the TCR signaling pathway. Thus, upon stimulation with a small number of antigens, a fully activated state occurs, thereby leading to a killing efficacy on the target cells. Compared with CAR-T, these TCR-T cells maintain and use all the accessory molecules in the TCR signaling pathway, thus the TCR-T is more sensitive in recognizing an antigen with low concentration and less copy number than some certain CAR-T, and it has very large therapeutic potential.

In some embodiments, TCR-T cells increase the affinity of the TCR for the corresponding antigen (e.g., TAA) by partial genetic modification, and the "genetically modified TCR" technique is therefore referred to as the "affinity enhanced TCR" technique. For example, a "genetically modified TCR" jointly developed by Adaptimmune Company (reported by Nature Medicine magazine) has several modified key amino acids. The affinity for the common cancer TAA, NY-ESO of these genetically modified TCR cells are greatly improved. This TCR may be used to fight cancers with overexpressed NY-ESO-1, such as multiple myeloma.

Adoptive cell therapy (ACT) or adoptive immunotherapy, such as tumor adoptive immunotherapy, refers to a therapeutic treatment for killing target cells (such as tumor cells), in which the immune cells are treated *in vitro* by, for instance, adding specific antigens, modifying the molecules they expressed, or stimulating with cytokines, screening and massively amplifying for the immune effector cells that specifically kill target cell (such as tumor cell), and then infused to patients. It is a kind of passive immunotherapy.

### Method for efficient T cells editing

In one aspect of the invention, provided is a method for preparing a genetically modified T cell (such as a universal T cell), comprising disrupting the following genomic regions in the T cell by genome editing technology: (i) the TRAC genomic region from base 23016448 to 23016490 on chromosome 14 (represented by SEQ ID NO: 23); (ii) the B2M genomic region from base 45003745 to 45003788 on chromosome 15 (represented by SEQ ID NO: 24); and/or (iii) the PD-1 genomic region from base 242800936 to 242800978 on chromosome 2 (represented by SEQ ID NO: 25), or the PD-1 genomic region from base 242795009 to 242795051 on chromosome 2 (represented by SEQ ID NO: 26). In some embodiments, the genome editing technology of the method is a zinc finger nuclease-based genome editing technology, a TALEN genome editing technology, or a CRISPR/Cas genome editing technology. In some embodiments, the TRAC genomic region, the B2M genomic region, or the PD-1 genomic region is edited. In some embodiments, wherein the TRAC genomic region, and the B2M genomic region are both edited. In some embodiments, wherein the TRAC genomic region and the PD-1 genomic region are both edited. In some embodiments, wherein the B2M genomic region and the PD-1 genomic region are both edited. In some embodiments, wherein all of the TRAC genomic region, the B2M genomic region, and the PD-1 genomic region are edited.

In some embodiments, provided is a method for preparing a genetically modified T cell (such as a universal T cell), comprising disrupting the following nucleotide sequences in the T cell by genome editing technology: (i) a TRAC genomic target nucleotide sequence complementary to a sequence selected from any one of SEQ ID NOs: 2-5; (ii) a B2M genomic target nucleotide sequence complementary to a sequence selected from any one of SEQ ID NOs: 6-14; and/or (iii) a PD-1 genomic target nucleotide sequence complementary to a sequence selected from any one of SEQ ID NOs: 15-22. In some embodiments, the genome editing technology of the method is a zinc finger nuclease-based genome editing technology, a TALEN genome editing technology, or a CRISPR/Cas genome editing technology. In some embodiments, the TRAC genomic region, the B2M genomic region, or the PD-1 genomic region is edited. In some embodiments, wherein the TRAC genomic region, and the B2M genomic region are both edited. In some embodiments, wherein the TRAC genomic region and the PD-1 genomic region are both edited. In some embodiments, wherein the B2M genomic region and the PD-1 genomic region are both edited. In some embodiments, wherein all of the TRAC genomic region, the B2M genomic region, and the PD-1 genomic region are edited.

In some embodiments, provided is a method for preparing a genetically modified T cell (such as a universal T cell), comprising: introducing a TRAC-targeted sgRNA, a B2M-targeted sgRNA, and/or a PD-1 sgRNA into the T cell, so as to destroy the TRAC, B2M and/or PD-1 gene of the T cell. In some embodiments, the method comprises introducing Cas9 or its encoding nucleotide sequence into the T cell.

In some embodiments, the sgRNA targets a gene encoding the constant region of α and/or β chain of TCR2, thereby disrupting the structure of the TCR on the surface of the T cell, and making the molecule to lose its function.

In some embodiments, the sgRNA targets a gene encoding β2-microglobulin (B2M), for example, targeting the first exon region of the B2M protein-encoding gene, thereby disrupting the structure of the B2M, and making the molecule to lose its function.

In some embodiments, the sgRNA targets a gene encoding PD-1, such as the first exon region of the PD-1 protein-encoding gene, thereby disrupting the structure of PD-1, and making the molecule to lose its function.

In some embodiments, provided is a method for preparing a genetically modified T cell (such as a universal T cell), comprising: (i) introducing a sgRNA comprising a sequence selected from any one of SEQ ID NOs: 2-5 into the T cell for editing the TRAC genomic region; (ii) introducing a sgRNA comprising a sequence selected from any one of SEQ ID NOs: 6-14 into the T cell for editing the B2M genomic region; and/or (iii) introducing a sgRNA comprising a sequence selected from any one of SEQ ID NOs: 15-22 into the T cell for editing the PD-1 genomic region. In some embodiments, the method comprises introducing Cas9 or its encoding nucleotide sequence into the T cell.

In some embodiments, the invention relates to introducing any TRAC-targeted sgRNA comprising a targeting sequence selected from Table 2 into the T cell. In some embodiments, the invention relates to introducing any B2M-targeted sgRNA comprising a target sequence selected from Table 2 into the T cell. In some embodiments, the invention relates to introducing any PD-1-targeted sgRNA comprising a target sequence selected from Table 2 into the T cell. In some embodiments, the method comprises introducing Cas9 or its encoding nucleotide sequence into the T cell.

In some embodiments, the invention relates to introducing any TRAC-targeted sgRNA comprising a target sequence selected from Table 2, and/or any B2M-targeted sgRNA comprising a target sequence selected from Table 2, and/or any PD-1-targeted sgRNA comprising a target sequence selected from Table 2, and Cas9 or its encoding nucleotide sequence into the T cell.

In some embodiments, the invention relates to introducing any TRAC-targeted sgRNA comprising a target sequence selected from Table 2, any B2M-targeted sgRNA comprising a target sequence selected from Table 2, and any PD-1-targeted sgRNA comprising a target sequence selected from Table 2 into the T cell. In some embodiments, the method comprises introducing Cas9 or its encoding nucleotide sequence into the T cell.

In some embodiments of the invention, provided is a method for preparing a universal T cell, comprising: (i) introducing a TRAC-sg3 sgRNA into the T cell for editing the TRAC genomic region; (ii) introducing a B2M-sg2 sgRNA into the T cell for editing the B2M genomic region; and (iii) introduction a PD-1-sg2 sgRNA into the T cell for editing the PD-1 genomic region. In some embodiments, the method comprises introducing Cas9 or its encoding nucleotide sequence into the T cell.

In some embodiments, provided is a method for preparing a universal T cell, comprising: (i) introducing a sgRNA comprising the sequence of SEQ ID NO: 2 or 3 into the T cell for editing the TRAC genomic region; (ii) introducing a sgRNA comprising a sequence selected from any one of SEQ ID NO: 7 or 11 into the T cell for editing the B2M genomic region; and (iii) introducing a sgRNA comprising a sequence selected from SEQ ID NO: 14 or 15 into T cells for editing the PD-1 genomic region. In some embodiments, the method comprises introducing Cas9 or its encoding nucleotide sequence into the T cell.

In some embodiments, the sgRNA described above is chemically modified. For example, it is a 2'-O-methyl sgRNA analog and/or internucleotide 3'-thio sgRNA. In some embodiments, the sgRNA has 2'-O-methyl nucleotide analogs on the first one, two, and/or three base(s) of the 5' end and/or the last base of the 3' end.

In general, a guide sequence in a sgRNA is any polynucleotide sequence sufficiently complementary to a target polynucleotide sequence so as to hybridize to the target sequence and direct the sequence-specific binding of a CRISPR complex to the target sequence. In some embodiments, when optimal alignment is performed by using an appropriate alignment algorithm, the degree of complementarity between a guide sequence and its corresponding target sequence is: about or greater than 80%, 85%, 90%, 95%, 97.5%, 99% or more. Optimal alignment can be determined by using any suitable algorithm for sequences alignment, and the non-limiting examples include: the Smith-Waterman algorithm, the Needleman-Wimsch algorithm, the Burrows-Wheeler Transform-based algorithm (e.g., Burrows Wheeler Aligner), ClustalW, Clustai X, BLAT, Novoalign (Novocraft Technologies), ELAND (Illumina, San Diego, CA), SOAP (available at soap.genomics.org.cn) and Maq (available at maq.sourceforge.net). In some embodiments, the length of the guide sequence may be about or more than about 10, 11, 12, 13, 14, 15, 16, 17, 18, 19, 20, 21, 22, 23, 24, 25, 26, 27, 28, 29, 30, 35, 40, 45, 50, 55, 60, 65, 70, 75 or more nucleotides. In some embodiments, the length of the guide sequence is less than about 75, 70, 65, 60, 55, 50, 45, 40, 35, 30, 25, 20, 15, 12 or fewer nucleotides. The ability of the guide sequence to direct sequence-specific binding of the CRISPR complex to the target sequence is assessed by any suitable assay. For example, a host cell having a corresponding target sequence can be provided with components of the CRISPR system (including the guide sequence to be tested) sufficient to form a CRISPR complex, such as a transfected vector encoding a CRISPR component. Then the preferential cleavage within the target sequence is evaluated. Similarly, the cleavage of a target polynucleotide sequence can be evaluated by providing the target sequence and the components of CRISPR complex (including the guide sequence to be tested and a control guide sequence different from the guide sequence) in a test tube, and then comparing the binding or cleavage rates of the tested and control guide sequences to the target sequence. The above assays and evaluations can also be carried out by using other assay methods known to those skilled in the art.

In some embodiments, the TRAC-targeted sgRNA, the B2M-targeted sgRNA, and/or the PD-1-targeted sgRNA and/or a Cas9-encoding nucleotide sequence (e.g., mRNA) are introduced into the T cell by electroporation, for example, introducing into the T cell under any of the following electroporation conditions: 150-250V, 0.5-2ms; 180-250V, 0.5-2ms; 150V, 2ms; 160V, 2ms; 170V, 2ms; 180V, 2ms; 190V, 1ms; 200V, 1ms; 210V, 1ms; 220V, 1ms; 230V, 1ms; 240V, 1ms; 250V, 0.5ms. In some embodiments, the TRAC-targeted sgRNA, the B2M-targeted sgRNA, the PD-1-targeted sgRNA, and a Cas9-encoding nucleotide sequence are co-introduced into the T cell by electroporation.

In some embodiments, the Cas9-encoding nucleotide sequence is an mRNA, such as an mRNA comprising an ARCA (Anti-Reverse Cap Analog). In some embodiments, the Cas9-encoding nucleotide sequence is in a viral vector, such as a lentiviral vector. In some embodiments, the Cas9-encoding nucleotide sequence comprises the sequence represented by SEQ ID NO: 1. In some embodiments, the TRAC-targeted sgRNA, the B2M-targeted sgRNA, and/or the PD-1-targeted sgRNA together with a Cas9-encoding nucleotide sequence are in the same vector.

In some embodiments, the TRAC-targeted sgRNA, the B2M-targeted sgRNA, and the PD-1-targeted sgRNA are simultaneously introduced into the T cell. In a particular embodiment, the TRAC-targeted sgRNA, the B2M-targeted sgRNA, and the PD-1-targeted sgRNA are simultaneously introduced into T cells, wherein each of the amount of the TRAC-targeted sgRNA, the B2M-targeted sgRNA, and the PD-1-targeted sgRNA can be approximate or equivalent. In some embodiments, the TRAC-targeted sgRNA, the B2M-targeted sgRNA, and the PD-1-targeted sgRNA are introduced into the T cell one by one in any appropriate order. In some embodiments, the TRAC-targeted sgRNA, the B2M-targeted sgRNA, and/or the PD-1-targeted sgRNA are simultaneously introduced into the T cell together with a Cas9-encoding nucleotide sequence. In some embodiments, a Cas9-encoding nucleotide sequence is introduced into the T cell prior to the TRAC-targeted sgRNA, the B2M-targeted sgRNA, and/or the PD-1-targeted sgRNA. In some embodiments, the T cell comprises a Cas9-encoding nucleotide sequence or a Cas9 protein.

In some embodiments, the T cells are derived from a healthy person. In some embodiments, the T cells are derived from a patient, such as a cancer patient, for example, a cancer patient prior to chemotherapy or radiation therapy. In some embodiments, the T cells are derived from umbilical cord blood, bone marrow, or peripheral blood mononuclear cells (PBMC). In some embodiments, the T cells are derived from stem cells, such as hematopoietic stem cells at various stages of differentiation. The preparation methods described herein may be used to knock out TRAC, B2M and/or PD-1 in, for example, PBMC or stem cells, and further culture, differentiate, and/or purify the corresponding-gene-modified T cells.

Compared with the prior art, the method for gene knockout in a T cell according to the present invention achieves a high efficiency of gene knockout, for example, the knockout efficiency of the knockout of a single gene (TRAC), double genes (TRAC and B2M), and triple genes (TRAC, B2M and PD-) is as high as at least 90%, 81% and 67% respectively.

"Knockout efficiency" can be expressed at the gene level by the efficiency of the INDEL which produces gene knockout, or at the cellular level by the percentage of cells in which the gene knockout causes the protein expressed by this gene to disappear or significantly decrease. In the present invention, "knockout efficiency" means the knockout efficiency calculated based on the latter. Those skilled in the art will appreciate that a high knockout efficiency can increase the harvest rate of the cells of interest, reducing the production costs and treatment costs.

In some embodiments, genetically modified T cells (e.g., universal T cells) are further screened to obtain higher purity T cells knocking out of a single gene (TRAC), double genes (TRAC and B2M), and three genes (TRAC, B2M, and PD- 1). For example, genetically modified T cells (such as universal T cells) with low expression levels of TRAC, B2M and/or PD-1 can be screened out by FACS.

In some embodiments, the TCR, and/or the HLA, and/or the PD-1 genes of the universal T cells according to the invention are knocked out.

In some particular embodiments, the gene coding the constant region (i.e., TRAC) of α chain of the TCR is knocked out. The coding region of B2M and/or PD-1 is knocked out. TRAC, B2M, and PD-1 can all be knocked out, or one of the three can be knocked out, or two of them can be knocked out.

In some particular embodiments, the gene coding the constant region (i.e., TRAC) of α chain of the TCR is knocked out. For example, in a particular embodiment, the gene of the constant coding region of TCR α chain according to the invention is knocked out by any one of the TRAC-sg 2, 3, 4, 6 molecules (see Table 2) and the Cas9 molecule which are introduced into the cell. Preferably, the gene of the constant region of TCR α chain is knocked out by the TRAC-sg2 or TRAC-sg3 and the Cas9 molecule which are introduced into the cell.

In other particular embodiments, the conserved coding region of B2M gene of HLA is knocked out. For example, in a particular embodiment, the conserved coding region of B2M gene is knocked out by any one of the B2M-sg 1-8 molecules (see Table 2) and the Cas9 molecule which are introduced into the cell. Preferably, the conserved coding region of B2M gene is knocked out by the B2M-sg2 or B2M-sg6 molecule and the Cas9 molecule which are introduced into the cell.

In other particular embodiments, the conserved coding region of PD-1 gene is knocked out. For example, in a particular embodiment, the conserved coding region of PD-1 gene is knocked out by any one of the PD-1-sg1-2 or PD-1-sg4-10 molecules (see Table 2) and the Cas9 molecule which are introduced into the cell. Preferably, the conserved coding region of PD-1 gene is knocked out by the PD-1-sg1 or PD-1-sg2 molecule and the Cas9 molecule which are introduced into the cell.

In other particular embodiments, the gene coding the constant region (i.e., TRAC) of α chain of the TCR and the B2M gene are knocked out. For example, in a particular embodiment of the invention, the TRAC and B2M genes are knocked out by the TRAC-sg3 and B2M-sg2 molecules (see Table 2) and the Cas9 molecule which are introduced into the cell.

In other particular embodiments, the gene coding the constant region (i.e., TRAC) of α chain of the TCR, the B2M gene, and the conserved coding region of PD-1 gene are knocked out. For example, in a particular embodiment of the invention, the TCR, HLA and the conserved coding region of PD-1 gene are knocked out by the TRAC-sg3, B2M-sg2 and PD-1-sg2 molecules (see Table 2) and the Cas9 molecule which are introduced into the cell. In a particular embodiment of the invention, the TCR, HLA and the conserved coding region of PD-1 gene are knocked out by the TRAC-sg2, B2M-sg6 and PD-1-sg1 molecules (see Table 2) and the Cas9 molecule which are introduced into the cell. In a particular embodiment of the invention, the TCR, HLA and the conserved coding region of PD-1 gene are knocked out by the TRAC-sg2, B2M-sg6 and PD-1-sg2 molecules (see Table 2) and the Cas9 molecule which are introduced into the cell.

In some embodiments, the invention provides a method for efficiently editing a T cell, the method comprises the steps of:
introducing sgRNA molecules and a Cas9 molecule into the T cell.

In some embodiments, the sgRNA molecules comprise a targeting domain respectively complementary to a target region of the gene coding the constant region (i.e., TRAC) of α chain of the TCR, the B2M gene, and the conserved coding region of PD-1 gene.

In some embodiments, the sgRNA molecule refers to a nucleic acid sequence comprising a targeting domain complementary to a target region of the gene to be knocked out, and it recognizes the target DNA sequence and directs the Cas9 molecule to cleave the target site, thereby achieving one-step high efficiency (more than 85% knockout efficiency) of the knockout of a corresponding locus.

In some embodiments, the sequence of the targeting domain comprised in the sgRNA molecule is represented by one of the sequences listed in Table 2.

In some preferred embodiments, the sequences of the targeting domains are represented by T2, B3 and P1.

In some preferred embodiments, the sgRNA molecule and the mRNA encoding a Cas9 molecule are introduced into the T cell by electroporation technique.

In some particular embodiments, the T cells used in the above methods are derived from healthy humans, such as peripheral blood of healthy adults, or cord blood of healthy humans who are naturally delivered.

In some embodiments, a third aspect of the invention provides a sgRNA sequence (Table 2), sgRNA with which enjoys high editing efficiency after specific modification.

In some particular embodiments, a sgRNA is chemically synthesized and modified to have more stable and higher editing efficiency than that of the sgRNA obtained by conventional *in vitro* transcription (IVT). Preferably, the T cell is subjected to a single electroporation, and the genome editing efficiency of chemically synthesized and modified sgRNA is 10 or more times than that of the sgRNA obtained by conventional IVT.

In some embodiments, provided is the use of the universal T cell of the invention in the preparation of a medicament for treating a disease (such as a tumor).

In some particular embodiments, the gene coding the constant region (i.e., TRAC) of α chain of the TCR, the conserved coding region of B2M gene of the HLA, and the conserved coding region of PD-1 gene are knocked out. For example, in a particular embodiment, the gene coding the constant region of α chain of the TCR according to the invention is knocked out by the TRAC-sg2 molecule introduced into the cell, the conserved coding region of B2M gene according to the invention is knocked out by the B2M-sg6 molecule introduced into the cell, the conserved coding region of PD-1 gene according to the invention is knocked out by the PD-1-sg1 molecule (see Table 2) introduced into the cell, and the Cas9 molecule; preferably, the gene coding the constant region of α chain of the TCR is knocked out by the TRAC-sg2 molecule introduced into the cell, the conserved coding region of B2M gene is knocked out by the B2M-sg6 molecule introduced into the cell, the conserved coding region of PD-1 gene is knocked out by the PD-1-sg1 molecule introduced into the cell, and the Cas9 molecule.

### Efficiently edited T cells or universal T cells

The present invention relates to TRAC single gene knockout T cells (TRAC^{negative}), TRAC/B2M double-gene knockout (DKO) T cells, and TRAC/B2M/PD-1 triple-gene knockout (TKO) T cells prepared by the above methods of the present invention.

Compared to the prior art, the gene knockout efficiency of the single gene knockout T cells (TRAC^{negative}), TRAC/B2M double-gene knockout (DKO) T cells, and TCR/B2M/PD-1 triple-gene knockout (TKO) T cells of the present invention is greatly improved.

The single gene knockout T cells (TRAC^{negative}), TRAC/B2M double-gene knockout (DKO) T cells, and TCR/B2M/PD-1 triple-gene knockout (TKO) T cells prepared by the invention can be precursor T cells to be further modified, or universal T cells for the preparation of various genetically modified T cells, for example, CAR-T cells or TCR-T cells.

In some embodiments of the invention, provided is a genetically modified T cell (such as a universal T cell), wherein in the T cell: (i) one or more loci in the TRAC genomic region from base 23016448 to 23016490 on chromosome 14 are disrupted by genome editing technology; (ii) one or more loci in the B2M genomic region from base 45003745 to 45003788 on chromosome 15 are disrupted by genome editing technology; and/or (iii) one or more loci in the PD-1 genomic region from base 242800936 to 242800978 on chromosome 2, or the PD-1 genomic region from base 242795009 to 242795051 on chromosome 2 are disrupted by genome editing technology. In some embodiments, in the T cell: (i) the TRAC genomic target nucleotide sequence complementary to a sequence selected from any one of SEQ ID NOs: 2-5 is disrupted by genome editing technology; (ii) the B2M genomic target nucleotide sequence complementary to a sequence selected from any one of SEQ ID NOs: 6-14 is disrupted by genome editing technology; and/or (iii) the PD-1 genomic target nucleotide sequence complementary to a sequence selected from any one of SEQ ID NOs: 15-22 is disrupted by genome editing technology.

In some embodiments of the invention, provided is a genetically modified T cell (such as a universal T cell), wherein in the T cell:
(i) the TRAC gene comprises any one of the sequences listed in Tables D and E;
(ii) the B2M gene comprises any one of the sequences listed in Tables B and C; and/or
(iii) the PD-1 gene comprises any one of the sequences listed in Table F or Table G.

In one aspect, the invention provides a composition comprising the genetically modified T cell (e.g., universal T cell), CAR-T cell, or TCR-T cell, such as a pharmaceutical composition.

In one aspect, the invention also provides a kit or a product comprising the genetically modified T cell (e.g., universal T cell) of the invention. The kit or the product may be used to prepare a CAR-T, TCR-T or other adoptive cell therapeutic composition.

### Method for preparing CAR-T cells

In one aspect, the invention provides a method for preparing a CAR-T cell, such as a universal CAR-T cell. In some embodiments, the method comprises introducing a CAR or its encoding nucleotide sequence or a vector thereof into any of the genetically modified T cells (e.g., universal T cells) described herein.

In some embodiments, provided is a method for preparing a CAR-T cell, comprising:
(i) introducing a sgRNA comprising a sequence targeting the TRAC gene from base 23016448 to 23016490 on chromosome 14 into the T cell to disrupt the TRAC genomic region; and/or
(ii) introducing a sgRNA comprising a sequence targeting the B2M genomic region from base 45003745 to 45003788 on chromosome 15 into the T cell to disrupt the B2M genomic region; and/or
(iii) introducing a sgRNA comprising a sequence targeting the PD-1 genomic region from base 242800936 to 242800978 on chromosome 2, or the PD-1 genomic region from base 242795009 to 242795051 to the T cell to disrupt the B2M genomic region; and
(iv) introducing a chimeric antigen receptor (CAR) or its coding nucleotide sequence into the T cell.

CAR or its coding nucleotide sequence, and the TRAC-targeted sgRNA, the B2M-targeted sgRNA and/or the PD-1-targeted sgRNA, and the CAR or its encoding nucleotide sequence can be introduced into the T cell in any appropriate order. In some embodiments, the TRAC-targeted sgRNA, the B2M-targeted sgRNA and/or the PD-1-targeted sgRNA, and CAR or its encoding nucleotide sequence are simultaneously introduced into the T cell. In some embodiments, the CAR or its encoding nucleotide sequence is introduced into the T cell prior to the TRAC-targeted sgRNA, the B2M-targeted sgRNA and/or the PD-1-targeted sgRNA. In some embodiments, the CAR or its encoding nucleotide sequence is introduced into the T cell that has been genetically modified, and the TRAC, B2M and/or PD-1 genomic regions of the T cell have been destroyed by editing. In some embodiments, the method further comprises introducing Cas9 or its encoding nucleotide sequence together with said sgRNA into the T cell.

In some embodiments, the CAR expressed by a universal CAR-T cell of the invention may be any CAR known in the art, as long as it enables a T cell to recognize the cell surface antigens in a human leukocyte antigen-independent manner, and exhibit a killing efficacy. For example, the CAR disclosed in the U.S. Patent Application US20140271635A1 can be used, and the CAR used in the invention may refer to the disclosure of this Patent Application US20140271635A1. In some embodiments, the CAR in a CAR-T cell of the invention is a CAR recognizing the antigens of *The Tumors and the Related Antigens* listed in the following Table A.

**Table A**

| **Antigens** | **Target tumors** |
|---|---|
| CD3 | T-cell lymphoma |
| CD5 | T-cell lymphoma |
| CD7 | T-cell lymphoma |
| CD20 | B-cell lymphoma, chronic lymphocytic leukemia |
| CD19 | precursor B-cell acute lymphoblastic leukemia, B-cell lymphoma, chronic lymphocytic leukemia |
| CD22 | precursor B-cell acute lymphoblastic leukemia, B-cell lymphoma, chronic lymphocytic leukemia |
| CD30 | Hodgkin's lymphoma, anaplastic large cell lymphoma |
| CD52 | T-cell acute myeloid leukemia, precursor B-cell acute lymphoblastic leukemia |
| CD70 | Hodgkin's lymphoma, diffuse large cell lymphoma, renal cell carcinoma, EBV + glioblastoma, undifferentiated nasopharyngeal sarcoma |
| CD33 | acute myeloid leukemia, myelodysplastic syndrome, acute promyelocytic leukemia, chronic myelogenous leukemia, myelomonocytic leukemia, acute lymphocytic leukemia (only 18%) |
| CD47 | precursor B-cell acute lymphoblastic leukemia, T-cell acute |
| | myeloid leukemia, acute myeloid leukemia |
| IL-7 receptor α | precursor B-cell acute lymphoblastic leukemia, B-cell lymphoma |
| Recombinant human thymic stromal lymphopoietin receptor | precursor B-cell acute lymphoblastic leukemia (7%), precursor B-cell acute lymphoblastic leukemia with Down syndrome (60%) |
| Receptor-like tyrosine kinase orphan receptor 1 | precursor B-cell acute lymphoblastic leukemia, chronic lymphocytic leukemia, mantle cell lymphoma |
| Ganglioside 2 | neuroblastoma, osteosarcoma, bone Ewing sarcoma, soft tissue sarcoma, melanoma |
| Interleukin 13 receptor α2 | glioblastoma, diffuse endothelial glioma, melanoma, various sarcomas, mesothelioma |
| Vascular endothelial growth factor receptor 2 | tumor vessels |
| Human epidermal growth factor receptor-2 | osteosarcoma, colon cancer, breast cancer |
| Anaplastic lymphoma kinase | neuroblastoma, neuroectodermal tumor, glioblastoma, rhabdomyosarcoma, melanoma |
| Epidermal growth factor receptor VIII | Glioma |
| Fibroblast growth factor receptor 4 | Rhabdomyosarcoma |
| B7-H3 | Neuroblastoma |
| Glypican-3 /Glypican-5 | nephroblastoma, neuroblastoma, rhabdomyosarcoma, liver cancer, melanoma |
| Folate receptor 1 | rhabdomyosarcoma, osteosarcoma |

In some embodiments, the CAR expressed in a CAR-T cell of the invention comprises a signal peptide, an extracellular binding region, a hinge region, a transmembrane region, and an intracellular signaling region. They are linked sequentially. The term "signal peptide" as used herein refers to a short (e.g., 5-30 amino acids in length) peptide chain that directs the transfer of a newly synthesized protein to a secretory pathway. In the present invention, a signal peptide of various proteins in a human body such as a signal peptide of a cytokine protein secreted *in vivo* or a leukocyte differentiation antigen (CD molecule) may be used.

In some embodiments, the signal peptide is a CD8 signal peptide, for example, its amino acid sequence is shown in the patent application US20140271635A1.

In some embodiments, the used hinge region is a hinge region of a variety of different antibodies or antigen receptors, particularly a hinge region of a CD molecule. In a particular embodiment, the hinge region may be selected from the hinge regions of proteins such as CD8 and CD28. The CD8 or CD28 is a natural marker on the surface of a T cell.

In the present invention, transmembrane regions of various human proteins may be used, particularly transmembrane regions of various antigen receptors. The transmembrane region preferably used is the transmembrane region of a CD molecule. In one embodiment, the transmembrane region may be selected from transmembrane regions of a protein such as CD8, CD28, and 4-1BB.

In some embodiments, the hinge region is a CD8α hinge region (CD8-hinge), and its amino acid sequence is shown in the patent application US20140271635A1.

The "extracellular binding region" refers to a region comprising a region that specifically recognizes a target antigen. In some embodiments, the extracellular binding region comprises a region that specifically recognizes a target tumor cell surface antigen. For example, this region can be an antigen-binding fragment of a scFv or other antibodies. The term "scFv" as used herein refers to a recombinant protein of a variable region of a heavy chain (VH) and a variable region of a light chain (VL) linked by a linker. The linker associates the two regions to ultimately form an antigen binding site. The scFv is typically an amino acid sequence encoded by a single nucleotide chain. The scFv described above may also include the derivatives thereof.

The CAR and its various domains used in the present invention may be further modified by conventional techniques known in the art, such as amino acid deletion, insertion, substitution, addition, and/or recombination, and/or other modification methods, either alone or in combination. Methods for introducing such modifications into the DNA sequences based on the amino acid sequence of an antibody are well known to those skilled in the art (see, for example, Molecular Cloning: A Laboratory Manual, by Sambrock et al., Cold Spring Harbor Laboratory (1989) N.Y.). The modification is preferably carried out at nucleic acid level.

The term "specifically recognize" as used herein means that the antigen recognition region of the invention does not or substantially does not cross-react with any polypeptide other than the antigen of interest. The degree of specificity can be determined by immunological techniques including, but not limited to, immunoblotting, immunoaffinity chromatography, flow cytometry, and the like.

In some embodiments, the extracellular binding region comprises an antigen binding region (such as a scFv) that specifically recognizes CD19, CEA, EGFR, GD2, CD7, CD138, or the like.

In some embodiments, the extracellular binding region comprises a humanized scFv that specifically recognizes CD19. In some embodiments, the amino acid sequence of the scFv specifically recognizing CD19 is that shown in the patent application US20140271635A1.

In the present invention, intracellular signal regions of various human body proteins, particularly intracellular signal regions of various antigen receptors, are used. The intracellular signal region preferably used is the intracellular signal region of a CD molecule. In a particular embodiment, the intracellular signal region is selected from the intracellular signal regions of CD3ζ, FcεRIγ, CD28, CD137 (4-1BB), CD134 protein, and their combinations thereof. The CD3 molecule consists of five subunits, wherein the CD3ζ subunit (also known as CD3zeta, ζ for short) contains three ITAM motifs, which are important signal transduction regions in the TCR-CD3 complex. FcεRIγ is mainly distributed on the surface of mast cells and basophils, and it contains an ITAM motif, the structure, distribution and function of which are similar to CD3ζ. In addition, as mentioned above, CD28, CD137, and CD134 are costimulatory signaling molecules, and the costimulation of the intracellular signal segments after binding to their respective ligands causes sustained proliferation of T cells, and enhances the levels of cytokines such as IL-2 and IFN-γ secreted by T cells, while increasing the survival time and anti-tumor effect of CAR-T cells *in vivo.*

In some embodiments, the signal produced by the TCR alone is insufficient to fully activate native T cells., and the sequence initiating antigen-dependent initial activation through TCR (primary intracellular signaling domain) and the sequence providing costimulatory signal in an antigen-independent manner (co-stimulatory domain) are needed. The primary signaling domain regulates the initial activation of the TCR complex in an irritant manner or in an inhibitory manner. The primary intracellular signaling domain acting in an irritating manner may contain a signaling motif, called the immunoreceptor tyrosine-based activation motif (ITAM). Examples of ITAM-containing primary intracellular signaling sequences suitable for use in the present invention include CD3ζ, FcRγ, FcRβ, CD3γ, CD3δ, CD3ε, CD5, CD22, CD79a, CD79b, and CD66d. In one embodiment, the primary signaling domain comprises a modified ITAM domain, such as a mutant ITAM domain with altered (e.g., increased or decreased as compared to a native ITAM domain) activity, or a primary intracellular signaling domain with a truncated ITAM. In one embodiment, the primary signaling domain comprises one or more ITAM motifs.

The costimulatory signaling domain refers to the part of the TCR that contains the intracellular domain of the costimulatory molecule. A costimulatory molecule is a cell surface molecule required besides antigen receptors or their ligands for the efficient reaction of a lymphocyte to an antigen. Examples of such molecules comprise CD27, CD28, 4-1BB (CD137), OX40, CD30, CD40, PD1, ICOS, lymphocyte function associated antigen-1 (LFA-1), CD2, CD7, LIGHT, NKG2C, B7-H3, ligands specifically binding to CD83, and the like.

In some embodiments, the invention provides a method for preparing a CAR-T cell, such as a universal CAR-T cell, the method comprises the steps of:

### 1) introducing sgRNA molecules and Cas9 molecules into the T cell;

In some embodiments, the sgRNA molecules comprise a targeting domain complementary to a target region from the gene coding the constant region (i.e., TRAC) of α chain of the TCR, the conserved coding region of B2M gene of HLA, and/or the conserved coding region of PD-1 gene.

### 2) introducing a CAR molecule into the T cell;

In some embodiments, the sgRNA molecule refers to a nucleic acid sequence comprising a targeting domain complementary to a target region of the gene to be knocked out, and it recognizes the target DNA sequence and directs the Cas9 molecule to cleave the target site, thereby achieving one-step high knockout efficiency (more than 85% knockout efficiency) of the corresponding locus.

In some embodiments, the Cas9 molecule refers to a mRNA of a Cas9 which cleaves a target site under the guidance of sgRNA.

In some particular embodiments, the sequence of the targeting domain comprised in the sgRNA molecule is represented by one of the sequences listed in Table 2.

In some preferred embodiments, the sequences of the targeting domains are shown as T2, B3, and P1 (Table 2).

In some preferred embodiments, the sgRNA molecules and the mRNA encoding a Cas9 molecule are introduced into the T cell by electroporation technique.

In some embodiments, the CAR molecule is introduced into the T cell by, for example, a lentiviral transfection technique.

In some particular embodiments, the method includes the steps of isolating and/or activating T cells from healthy human peripheral blood or cord blood; preferably, the method further comprises the steps of sorting the universal CAR-T cells after step 2) above; more preferably, the functions of the obtained CAR-T cells, such as universal CAR-T cells, are verified after sorting.

In some embodiments, the invention provides the use of the above CAR-T cells for the preparation of a medicament for treating a disease, such as a tumor.

In one aspect, the invention provides a method for treating a disease in a subject, the method comprises administering to the subject an effective amount of CAR-T cells of the invention. The diseases that the method described herein treats include, but are not limited to, cancer and HIV/AIDS. In some embodiments, the disease is a tumor, including a hematological tumor, such as a lymphoma or leukemia. In some embodiments, the CAR targets an antigen listed in Table A, and the disease is a tumor listed in Table A that corresponds to the target antigen. In some embodiments, the T cell is not obtained from a subject. For example, the T cells are derived from a healthy donor.

The CAR-T cells to which the present invention relates can be administered to a subject in need thereof by a route conventionally used for administering a pharmaceutical preparation containing a cellular component, such as an intravenous infusion route. The dosage of administration can be specifically determined according to the pathogenic and health conditions of the subject.

### Method for preparing TCR-T cells

The invention provides a T cell expressing engineered TCR, also known as a TCR-T cell. The invention also provides a method for preparing the TCR-T cell, wherein the method comprises introducing an engineered TCR or its coding nucleotide sequence or a vector thereof into any of the genetically modified T cells (e.g., universal T cells) described herein.

In some embodiments, provided is a method for preparing a TCR-T cell, comprising:
(i) introducing a sgRNA comprising a sequence targeting the TRAC gene from base 23016448 to 23016490 on chromosome 14 into the T cell to disrupt the TRAC genomic region; and/or
(ii) introducing a sgRNA comprising a sequence targeting the B2M genomic region from base 45003745 to 45003788 on chromosome 15 into the T cell to disrupt the B2M genomic region; and/or
(iii) introducing a sgRNA comprising a sequence targeting the PD-1 genomic region from base 242800936 to 242800978 on chromosome 2, or the PD-1 genomic region from base 242795009 to 242795051 to the T cell to disrupt the B2M genomic region; and
(iv) introducing an engineered TCR or its encoding nucleotide sequence into the T cell.

TCR or its coding nucleotide sequence, and the TRAC-targeted sgRNA, the B2M-targeted sgRNA and/or the PD-1-targeted sgRNA, and the TCR or its encoding nucleotide sequence can be introduced into the T cell in any appropriate order. In some embodiments, the TRAC-targeted sgRNA, the B2M-targeted sgRNA and/or the PD-1-targeted sgRNA, and TCR or its encoding nucleotide sequence are simultaneously introduced into the T cell. In some embodiments, the TCR or its encoding nucleotide sequence is introduced into the T cell prior to the TRAC-targeted sgRNA, the B2M-targeted sgRNA and/or the PD-1-targeted sgRNA. In some embodiments, the TCR or its encoding nucleotide sequence is introduced into the T cell that has been genetically modified, and the TRAC, B2M and/or PD-1 genomic regions of the T cell have been destroyed by editing. In some embodiments, the method further comprises introducing Cas9 or its encoding nucleotide sequence together with said sgRNA into the T cell.

In some embodiments, the engineered TCR expressed by the TCR-T cells of the invention may be any engineered TCR known in the art, as long as it enables a T cell to recognize the cell surface antigens in a human leukocyte antigen-independent manner, and exhibit a killing efficacy. For example, an engineered TCR in a TCR-T cell of the invention can be an engineered TCR that recognizes an antigen listed in Table A.

As used herein, "an engineered TCR molecule", "an engineered TCR molecule" or "an artificial TCR molecule" includes a recombinant polypeptide derived from various polypeptides making up a TCR, the recombinant polypeptide can generally i) bind to a surface antigen on the target cell; and ii) interact with other polypeptide components of a intact TCR complex when co-localized in or on the T cell.

In a particular embodiment, an engineered TCR of the invention comprises a target-specific binding element, also known as an antigen binding domain. A suitable antigen binding domain recognizes, for example, a target antigen acting as a cell surface marker on a target cell associated with a particular disease state. In a particular embodiment, the target antigen is, for example, an antigen listed in Table A above. In a particular embodiment, the target antigen is, for example, a target antigen associated with a viral infection or an autoimmune disease. By genetic engineering, the antigen binding domain can be combined with various polypeptides derived from the composition of the TCR, such that the TCR-mediated T cell response is directed against the antigen of interest.

In a particular embodiment, the engineered TCR of the invention comprises a transmembrane domain. The transmembrane domain can be derived from a natural source or a recombinant source. Wherein the source of a natural source, the domain may be derived from any membrane-anchored or transmembrane protein. In one aspect, the transmembrane domain transduces signals to an intracellular domain as long as the engineered TCR binds to the target. Particularly transmembrane domains suitable for the present invention may include, but not limited to for example, the transmembrane regions of a T cell receptor α, β, or ζ chain, CD28, CD3ε, CD45, CD4, CD5, CD8, CD9, CD16, CD22, CD33, CD37, CD64, CD80, CD86, CD134, CD137, and CD154.

In some embodiments, the transmembrane domain can be linked to the extracellular region of an engineered TCR, such as an antigen binding domain of an engineered TCR, by a hinge, such as a hinge from a human protein. For example, in one embodiment, the hinge can be a human immunoglobulin (Ig) hinge, such as an IgG4 hinge, or a CD8a hinge.

In a particular embodiment, an engineered TCR of the invention comprises a linker linking a transmembrane domain to a cytoplasmic region. Optionally, the linker is a short oligopeptide or polypeptide linker with 2-50 amino acids in length. Glycine-serine pairs provide a particularly suitable linker.

In a particular embodiment, the engineered TCR of the invention comprises a cytoplasmic domain. The intracellular signaling domain is generally responsible for the activation of at least one of the normal effector functions of the immune cells into which the engineered TCR is introduced. The effector function of T cells is, for example, a cytolytic activity or a helper activity, including the secretion of cytokines. Thus, the term "intracellular signaling domain" refers to a portion of a protein that transduces an effector function signal and directs the cell to perform a specific function. Although an intact intracellular signaling domain can generally be employed, in many cases it is not necessary to use a full chain, and the truncation may be used in place of the full chain as long as the truncation transduces the effector function signal. Thus, the term intracellular signaling domain is intended to include any truncation of an intracellular signaling domain sufficient to transduce an effector function signal.

In some embodiments, the engineered TCR molecule comprises an engineered TCRα and TCRβ chain. In some embodiments, the engineered TCR molecule combines to a CD3 molecule expressed in the T cell and a ζ chain and/or other costimulatory molecules.

In some embodiments, the invention provides the use of the above TCR-T cells for the preparation of a medicament for the treatment of a disease, such as a tumor.

In one aspect, the invention provides a method for treating a disease in a subject, the method comprises administering to the subject an effective amount of the TCR-T cell of the invention. The diseases the therapeutic methods described herein treat include, but are not limited to, cancer and HIV/AIDS. In some embodiments, the disease is a tumor, including a hematological tumor, such as a lymphoma or leukemia. In some embodiments, the engineered TCR targets a target antigen listed in Table A, the disease is a tumor listed in Table A and corresponds to the target antigen. In some embodiments, the T cell is not obtained from the subject. For example, the T cells can be derived from a healthy donor.

The TCR-T cells to which the present invention relates may be administered to a subject in need thereof by a route conventionally used for administering a pharmaceutical preparation containing a cellular component, such as an intravenous infusion route. The dosage administered can be specifically determined according to the pathogenic and health condition of the subject.

### T cell sources

The T cell source is obtained from a subject prior to amplification and genetic modification. The term "subject" is intended to include a living organism (e.g., a mammal) capable of conduct an immune response. Examples of the subjects include humans. T cells can be obtained from a variety of sources, including peripheral blood mononuclear cells, bone marrow, lymph node tissue, cord blood, thymus tissue, tissues from infected sites, ascites, pleural effusion, spleen tissue, and tumors. The T cells of the invention may also be derived from hematopoietic stem cells at various stages of differentiation. Hematopoietic stem cells differentiate into T cells under directed differentiation culture conditions. In some aspects of the invention, a variety of T cell lines available in the art may be used.

In some aspects of the invention, T cells can be obtained from blood collected from a subject by using a variety of techniques known to those skilled in the art, such as FicollTM isolation. The T cells can also be obtained from the individual's circulating blood by apheresis. The products of apheresis typically contain lymphocytes, including T cells, monocytes, granulocytes, B cells, other nucleated leukocytes, red blood cells, and platelets. In one aspect, the cells collected by apheresis can be washed to remove plasma fractions and placed in appropriate buffers or media for subsequent processing steps.

T cells can be isolated from peripheral blood lymphocytes by dissolving the red blood cells and depleting the monocytes, for example, by PERCOLL™ gradient centrifugation or countercurrent centrifugation. Specific T cell subsets, such as CD3+, CD28+, CD4+, CD8+, CD45RA+, and CD45RO+ T cells can be further isolated by positive or negative selection techniques. For example, in one aspect, T cells are separated by incubating with anti-CD3/anti-CD28 (e.g., 3x28) coupled beads, such as DYNABEADS™ M-450CD3/CD28T, for a time enough to positively select the desired T cells. Tumor infiltrating lymphocytes (TIL) can be isolated from a tumor tissue.

### sgRNA

In one aspect, the invention provides a TRAC-targeted sgRNA, a B2M-targeted sgRNA, and a PD-1-targeted sgRNA. The sgRNAs comprise any nucleotide sequence selected from SEQ ID NOs: 2-22. In some embodiments, the sgRNA is chemically modified.

The invention further includes a sgRNA composition, a kit, or a product, comprising a sgRNA of the invention or a vector thereof. In some embodiments, the kit comprises: i) a sgRNA comprising a sequence selected from any one of SEQ ID NOs: 2-5; (ii) a sgRNA comprising a sequence selected from any one of SEQ ID NOs: 6-14; and/or (iii) a sgRNA comprising a sequence selected from any one of SEQ ID NOs: 15-22. In some embodiments, the kit comprises: (i) a sgRNA comprising the sequence of SEQ ID NO: 3; (ii) a sgRNA comprising the sequence of SEQ ID NO: 16; and (iii) a sgRNA comprising the sequence of SEQ ID NO: 7. In some embodiments, the kit further comprises a Cas9-encoding nucleic acid or a vector thereof. In some embodiments, the sgRNA is chemically modified.

In some embodiments, a chemically modified sgRNA is used in the method for preparing a genetically modified T cell according to the invention. The chemically modified sgRNA employed in the present inventors is considered to have the following two advantages. Firstly, since the sgRNA is a single-stranded form of RNA, its half-life period is very short; when it enters the cell, it will degrade rapidly (the maximum life period is not more than 12 hrs.), and it takes at least 48 hrs. for Cas9 protein to bind to sgRNA for genome editing. Therefore, the chemically modified sgRNA is stable after entering the cell, and after binding to the Cas9 protein, the genome can be efficiently edited to generate indels. Secondly, an unmodified sgRNA has poor ability to penetrate cell membrane and cannot effectively enter a cell or tissue to function correspondingly. The ability of a chemically modified sgRNA to penetrate cell membrane is generally enhanced. The chemical modification methods commonly used in the art may be employed in the present invention, as long as the sgRNA stability (prolonged half-life) and the ability to enter the cell membrane can be improved. In addition to the specific chemical modifications used in the examples, other modification methods are also included, for example, the chemical modification methods reported in the following articles: Deleavey GF1, Damha MJ. Designing chemically modified oligonucleotides for targeted gene silencing. Chem Biol. 2012 Aug. 24; 19(8): 937 -54; and Hendel et al., Chemically modified guide RNAs enhance CRISPR-Cas genome editing in human primary cells. Nat Biotechnol. 2015 Sep; 33 (9): 985-989.

In the present invention, the chemically modified sgRNA and Cas9-encoding gene are co-introduced into a T cell by electroporation, resulting in efficient genome editing efficiency (e.g., indicating as Indels%), wherein chemical modification of the sgRNA is one of the key factors in the present invention. The data in the examples shows that if the chemically unmodified sgRNA and Cas9-encoding gene are co-introduced into a T cell by electroporation, the indels efficiency is much lower than that obtained from the electroporation by using a chemically modified sgRNA.

The contents of the present invention are further described below with specific Examples. It should be understood that the specific examples are only for illustrative purposes, and do not mean that the contents of the present invention are limited to the specific examples.

Several documents have been cited throughout the text of this specification. Each of the documents (including any journal articles or abstracts, published or unpublished patent applications, granted patents, manufacturer's specifications, instructions for use, etc.) is incorporated herein by reference in its entirety. However, it is not an admission that the documents cited herein are in fact prior art to the present invention.

### Example 1: Preparation of universal T cells

### 1. Isolation and activation of healthy donor-derived T cells

Collection of cord blood from healthy donors: firstly the umbilical cord blood is taken from the blood bank, and then put into the refrigerator at 4°C for temporary storage; within 24 hrs. it is transported to the GMP laboratory via a transport vehicle equipped with a constant temperature device, so as to the isolate T cells.
1.1 Preparation of cord blood mononuclear cells: the physiological saline is added by pipette into the cord blood transported in step (1), the cord blood and physiological saline are diluted according to a ratio of 1:1 (V/V), then the diluent of blood cell is slowly added into a tube for the separation of lymphocytes. After centrifuging at 800g for 20 min the cells in the buffy coat above the lymphocyte separation medium are pipetted and transferred to a new 50 ml centrifuge tube, then the T cell culture medium added. After centrifuging at 400g for 5 min, the supernatant is discarded, and the peripheral blood mononuclear cells are obtained by retaining the cell pellet at the bottom of the centrifuge tube.
1.2 Isolation and activation of T cells: the obtained cord blood mononuclear cells are counted by a cell counter, and then subjected to T cell sorting. The specific steps are as follows:
   1.2.1. The cell pellet is adjusted to a density of 5^{∗}10⁷/ml with Easy buffer (manufacturer: StemCell, Cat. No. 16F72331), and the cells are transferred to a 5 ml flow cytometry tube with a 5 ml pipette;
   1.2.2. T cell isolation medium is added at a concentration of 50 µl/ml, then incubating for 5 min at room temperature;
   1.2.3. Magnetic beads for sorting are added at a concentration of 40 µl/ml, then mixed within 30 s;
   1.2.4 The cell suspension is supplemented to 2.5 ml with Easy buffer, then directly placed on the magnetic column for 3 min, and the cells are poured into a 15 ml centrifuge tube to obtain T cells;
   1.2.5. After sorting, the cells are mixed by a 1000 µl pipette and counted. Then by centrifuging (400G, 5min) and discarding the supernatant, a T cell pellet is obtained.

The T cell pellet is re-suspended in the T cells culture medium. Then the T cell activating factor is added at a ratio of 1:1. When the T cells are activated, they are placed in an incubator for subsequent amplification.

### 2. Optimization of the electroporation conditions

The above cultured T cells are collected into a 50 ml centrifuge tube, centrifuging at 300g for 7 min, then the supernatant is discarded. The pellet is washed twice with DPBS solution (manufacturer: Gibco, Cat. No. 1924294), and then the cell density is adjusted to 2.5×10⁷ cells/mL with electroporation reagent. GFP mRNA prepared (particular steps referring to section 3.3 below) by using HISCRIBE™ T7 ARCA mRNA Kit (tailed) (manufacturer: NEB, Cat. No.: cat#E2060S) is well mixed with T cells to a final concentration of 2.5×10⁶ cells and 6 µg of GFP mRNA per 100 µl. Then GFP mRNA is introduced into the T cells by using BTX Agile pulse MAX electroporation system (manufacturer: BTX, model: 47-0200 NINT). Different voltages and pulse times are tested to optimize the electroporation system. As shown in Table 1, the voltage is successively increased from 180 volts to 400 volts, and the pulse time is successively decreased from 2 ms to 0.5 ms. The growth of the cells is observed every day. The T cells subjected to electroporation are counted and the medium is replaced every other day. Phenotypic analysis on the genetically modified T cells is performed by a flow cytometer (manufacturer: ACEABIO, model: ACEA NovoCyte), and the results are shown in Table 1. Under the electroporation conditions of 150-250 V and 0.5-2 ms, the cell viability and the GFP electroporation efficiency are the best.

**Table 1: Comparison on electroporation efficiency of GFP mRNA and cell viability after electroporation under different electroporation conditions.**

| Volts | Pulse (ms) | Interval (ms) | Pulse | Viability (%) | GFP(%) |
|---|---|---|---|---|---|
| 400 | 0.5 | 100 | 1 | 19.78 | 14.01 |
| 360 | 1 | 100 | 1 | 21.85 | 11.85 |
| 300 | 1 | 100 | 1 | 31.99 | 22.91 |
| 300 | 1 | 100 | 1 | 32.20 | 32.19 |
| 250 | 3.5 | 100 | 1 | 93.22 | 93.19 |
| 250 | 1 | 100 | 1 | 57.18 | 57.37 |
| 210 | 1 | 100 | 1 | 91.97 | 92.23 |
| 200 | 1 | 100 | 1 | 94.54 | 94.46 |
| 200 | 2 | 100 | 1 | 65.30 | 65.40 |
| 190 | 1 | 100 | 1 | 94.61 | 94.66 |
| 180 | 2 | 100 | 1 | 93.77 | 93.65 |

### 3. Gene knockout of T cells

The TRAC, B2M, and PD-1 genes in the T cells obtained in step 1.2 are knocked out by using a CRISPR/Cas9 genome editing technology. The specific steps are as follows:
3.1 The sgRNA design and plasmid construction are aimed to α chain constant region (i.e., TRAC) gene of TCR, the conserved coding region of B2M gene of HLA and the conserved coding region of PD-1 gene.
All the sgRNAs for the coding sequences of the TRAC, B2M and PD-1 coding regions are designed by CRISPR RGEN Tools. The sgRNA sequences selected according to the highest score are shown in Table 2.

**Table 2: Selected sgRNA sequences**

| | | |
|---|---|---|
| T2 | GCTGGTACACGGCAGGGTCA | SEQ ID NO: 2 |
| T3 | CTCTCAGCTGGTACACGGCA | SEQ ID NO: 3 |
| T4 | ATTTGTTTGAGAATCAAAAT | SEQ ID NO: 4 |
| T6 | TCTCTCAGCTGGTACACGGC | SEQ ID NO: 5 |
| B1 | ACTCTCTCTTTCTGGCCTGG | SEQ ID NO: 6 |
| B2 | GAGTAGCGCGAGCACAGCTA | SEQ ID NO: 7 |
| B3 | CGCGAGCACAGCTAAGGCCA | SEQ ID NO: 8 |
| B4 | TCACGTCATCCAGCAGAGAA | SEQ ID NO: 9 |
| B5 | GCTACTCTCTCTTTCTGGCC | SEQ ID NO: 10 |
| B6 | TTTGACTTTCCATTCTCTGC | SEQ ID NO: 11 |
| B7 | CGTGAGTAAACCTGAATCTT | SEQ ID NO: 12 |
| B8 | CTCGCGCTACTCTCTCTTTC | SEQ ID NO: 13 |
| P1 | CTGCAGCTTCTCCAACACAT | SEQ ID NO: 14 |
| P2 | GCCCTGGCCAGTCGTCTGGG | SEQ ID NO: 15 |
| P4 | GCCCTGCTCGTGGTGACCGA | SEQ ID NO: 16 |
| P5 | GAGAAGGTGGGGGGGTTCCA | SEQ ID NO: 17 |
| P6 | CCCTGCTCGTGGTGACCGAA | SEQ ID NO: 18 |
| P7 | GAAGGTGGCGTTGTCCCCTT | SEQ ID NO: 19 |
| P8 | CCTGCTCGTGGTGACCGAAG | SEQ ID NO: 20 |
| P9 | GTCTGGGCGGTGCTACAACT | SEQ ID NO: 21 |
| P10 | CGATGTGTTGGAGAAGCTGC | SEQ ID NO: 22 |

Wherein T represents the sgRNA sequences for TRAC, P represents the sgRNA sequences for the PD-1 coding region, and B represents the sgRNA sequences for B2M.
3.2 The sgRNA is a 2'-O-methyl sgRNA analog and/or an internucleotide 3'-thio sgRNA with high knockout efficiency and stability and is produced with chemical modification methods.
3.3 Cas9 plasmid and GFP plasmid are linearized by enzymatic digestion, using Xbal (manufacturer: NEB, Cat. No.: cat#R0145S), cutsmart buffer (manufacturer: NEB, Cat. No.: cat#B7204s) in 50µl reaction system:

| Components | Volumes |
|---|---|
| Cas9 plasmid | 5µg |
| 10^{∗}cutsmart buffer | 5µl |
| Xbal | 2µl |
| Adding water to the total reaction volume | 50µl |

After 4 hours in a water bath at 37°C, 2 µl of the enzyme-digested product is taken for agarose gel electrophoresis. The voltage is set to 110 U, and electrophoresis is carried out for 30 min. A single band is observed with a gel imager, indicating that the enzyme digestion is complete and all plasmids are linearized.
The above reaction product is washed and purified.
The purified product is subjected to *in vitro* transcription (i.e., IVT) by using a HISCRIBE™ T7 ARCA mRNA Kit (manufacturer: NEB, Cat. No.: cat#E2060S), in 20 µl system:

| Components | Volumes |
|---|---|
| 2^{∗}ARCA Mix | 10µl |
| Template DNA | 0.5µg |
| T7 RNA Polymerase Mix | 2µl |
| Nuclease-free water | adding to 20µl |
| Total reaction volume | 20µl |

The above reaction system is placed on a PCR amplifier at 37°C for 4 hrs. After 4 hours, 2 µl of DNase 1 is added to the reaction system, and the reaction is carried out at 37°C for 20 min.
The above reaction product is taken to proceed as follows:

| Components | Volumes |
|---|---|
| Nuclease-free water | 63µl |
| IVT reaction product | 22µl |
| 10^{∗}Poly(A) polymerase buffer | 10µl |
| Poly(A) polymerase | 5µl |
| Total reaction volume | 100µl |

The above reaction system is placed on a PCR amplifier for 2 hrs.
The above reaction product is washed and purified, then stored in a -80 °C refrigerator for use.
3.4 The sgRNA and Cas9 mRNA are introduced into T cells by electroporation technique, and the T cells with TCR and/or B2M and/or PD1 negative together with CD4 and CD8 positive are screened out by antigen screening principle to obtain universal T cells.
The above cells are taken for the genome extraction by using TIANamp Genomic DNA Kit (manufacturer: TIAN GEN, Cat. No.: cat# DP304-03). Synthetic primers, the above extracted cell genome, and 2^{∗}Easy Taq Super Mix (+dye) (manufacturer: TRANS, Cat. No. Code#AS111) are used to respectively PCR-amplify the genomic regions of TRAC, B2M and PD-1 including the corresponding sgRNA in a 50µl reaction system:

| Components | Volumes |
|---|---|
| 2^{∗}Easy Taq Super Mix(+dye) | 25µl |
| Primer F (10 pmol/µl) | 1µl |
| Primer R (10 pmol/µl) | 1µl |
| Template DNA | 300ng |
| Adding water to the total volume | 50µl |

The reaction conditions are as follows: 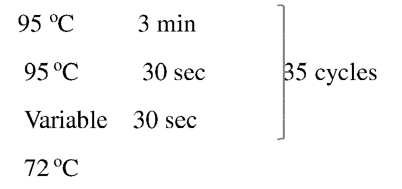
3.5 The reaction product is subjected to Sanger sequencing to verify the knockout efficiency of TCR, HLA and PD-1 at the molecular level. The results are shown in Figure 2. With this system, the TCR gene of TCR, B2M gene of HLA, PD-1 conserved coding sequence can be successfully edited by CRISPR/Cas9 technology, including insertion mutations and deletion mutations, both of which cause frameshift mutations (see Table B-G below), thereby inhibiting the expression of TCR, HLA, and PD-1 at gene level.

**Table B: Sequence changes produced from base 45003745 to 45003788 after the introduction of B2:**

| "-" means the deleted base after editing, "n" means the insertion of any base | Change in sequence length | Sequence numbers |
|---|---|---|
| ATGTCTCGCTCCGTGGCCTTAGCTGTGCTCGCGCT ACTCTCTCT | Original sequence | SEQ ID NO: 24 |
| ATGTCTCGCTCCGTGGCCTTA-CTGTGCTCGCGCTACTCTC TCT | -1 | SEQ ID NO: 27 |
| ATGTCTC-------------------------GCTACTCTCTCT | -25 | SEQ ID NO: 28 |
| ATGTCTCGCTCCGTGGCCTTAGnCTGTGCTCGCGCTACTC TCTCT | 1 | SEQ ID NO: 29 |
| ATGTCTCGCTCCGTGGCCTTAG---TGCTCGCGCTACTCTCT CT I | -3 | SEQ ID NO: 30 |
| ATGTCTCGCTCCGTGG-----------CTCGCGCTACTCTCTCT | -11 | SEQ ID NO: 31 |
| ATGTCTCGCTCCGTGG-------------------ACTCTCTCT | -19 | SEQ ID NO: 32 |
| ATGTCTCGCTCCGTGGC-----CTGTGCTCGCGCTACTCTCTC T | -5 | SEQ ID NO: 33 |
| ATGTCTCGCTCC-----------------------ACTCTCTCT | -23 | SEQ ID NO: 34 |
| ATGTCTCGCTCCGTGG---------------CGCTACTCTCTCT | -15 | SEQ ID NO: 35 |
| ATGTCTCGCTCCGTGG----------------GCTACTCTCTCT | -16 | SEQ ID NO: 36 |
| ATGTCTCGCTCCGTGGCCTTAGnnCTGTGCTCGCGCTACTC | 2 | SEQ ID NO: 37 |
| TCTCT | | |
| ATGTCTCGCTCCGTGG----------GCTCGCGCTACTCTCTCT | -10 | SEQ ID NO: 38 |
| ATGTCTCGCTCCGTGGCCT------------CGCTACTCTCTCT | -12 | SEQ ID NO: 39 |
| ATGTCTCGCTCCGTGGCCTTA--TGTGCTCGCGCTACTCTC TCT | -2 | SEQ ID NO: 40 |
| ATGTCTC------------------------------ TCTCTCT | -30 | SEQ ID NO: 41 |
| ATGTCTCGCTCCGTGGCC-----------------ACTCTCTCT | -17 | SEQ ID NO: 42 |
| ATGTCTCGCTCCGTGGCCTTAG----GCTCGCGCTACTCTCT CT | -4 | SEQ ID NO: 43 |
| ATGTCTCGCTCCGTGGCCTTAG--GTGCTCGCGCTACTCTC TCT | -2 | SEQ ID NO: 44 |
| ATGTCTCG-----------------TGCTCGCGCTACTCTCTCT | -17 | SEQ ID NO: 45 |
| ATGTCTCGCTCCGTGGC------------CGCGCTACTCTCTCT | -12 | SEQ ID NO: 46 |
| ATGTCTCGCTCCGTGGCCTTAG-----CTCGCGCTACTCTCTC T | -5 | SEQ ID NO: 47 |
| ATGTCTC-----------------------GCGCTACTCTCTCT | -23 | SEQ ID NO: 48 |
| ATGTCTCGCTCCGTGGC------------------ACTCTCTCT | -18 | SEQ ID NO: 49 |
| ATGTCTCGCTCCGTGGC-------GTGCTCGCGCTACTCTCTCT | -7 | SEQ ID NO: 50 |
| ATGTCTCGCTCCGTG--------------------ACTCTCTCT | -20 | SEQ ID NO: 51 |
| ATGTCTCGCT----------------------CTACTCTCTCT | -23 | SEQ ID NO: 52 |

| | | |
|---|---|---|
| Note: The "-" in the table is marked as "d" in the sequence listing. | | |

**Table C: Sequence changes produced on chromosome 15 from 45003745 to 45003788 after the introduction of B3:**

| "-" means the deleted base after editing, "n" means the insertion of any base | Change in sequence length | Sequence numbers |
|---|---|---|
| ATGTCTCGCTCCGTGGCCTTAGCTGTGCTCGCGCTACTCTCTCT | Original sequence | SEQ ID NO: 24 |
| ATGTCTCGCTCCGTGG-CTTAGCTGTGCTCGCGCTACTCTCTCT | -1 | SEQ ID NO: 53 |
| ATGTCTCGCTCCGTG--CTTAGCTGTGCTCGCGCTACTCTCTCT | -2 | SEQ ID NO: 54 |
| ATGTCTCGCTCCGTG------GCTGTGCTCGCGCTACTCTCTCT | -6 | SEQ ID NO: 55 |
| ATGTCTCGCTCCGTGG--TTAGCTGTGCTCGCGCTACTCTCTCT | -2 | SEQ ID NO: 56 |
| ATGTCT-----------------------CGCGCTACTCTCTCT | -23 | SEQ ID NO: 57 |
| ATGTCTCGCTCC------TTAGCTGTGCTCGCGCTACTCTCTCT | -6 | SEQ ID NO: 58 |
| ATGTCTCGCTCCGTG---TTAGCTGTGCTCGCGCTACTCTCTCT | -3 | SEQ ID NO: 59 |
| A----------------------TGTGCTCGCGCTACTCTCTCT | -22 | SEQ ID NO: 60 |
| ATGTCTCGCTCCGTG--------------CGCGCTACTCTCTCT | -14 | SEQ ID NO: 61 |
| ATGTCTC---------CCTTAGCTGTGCTCGCGCTACTCTCTCT | -9 | SEQ ID NO: 62 |
| ATGTC------------CTTAGCTGTGCTCGCGCTACTCTCTCT | -12 | SEQ ID NO: 63 |
| A-------------------------GCTCGCGCTACTCTCTCT | -25 | SEQ ID NO: 64 |
| ATGTCTCGCTCCGTG-----AGCTGTGCTCGCGCTACTCTCTCT | -5 | SEQ ID NO: 65 |
| A-----------------------GTGCTCGCGCTACTCTCTCT | -23 | SEQ ID NO: 66 |
| ATGTCTCGCTCCG------------TGCTCGCGCTACTCTCTCT | -12 | SEQ ID NO: 67 |
| ATGTCTCGCTCCGTGGnCCTTAGCTGTGCTCGCGCTACTCTCTCT | 1 | SEQ ID NO: 68 |
| ATGTCTCGCTCCGTG----TAGCTGTGCTCGCGCTACTCTCTCT | -4 | SEQ ID NO: 69 |
| A-------------------AGCTGTGCTCGCGCTACTCTCTCT | -19 | SEQ ID NO: 70 |
| ATGTCTCGCTCC----CCTTAGCTGTGCTCGCGCTACTCTCTCT | -4 | SEQ ID NO: 71 |
| ATGTCTCGCTC--------------------CGCTACTCTCTCT | -20 | SEQ ID NO: 72 |
| ATGTCTCGCTCC-------TAGCTGTGCTCGCGCTACTCTCTCT | -7 | SEQ ID NO: 73 |
| ATGTCTCGCT------------------TCGCGCTACTCTCTCT | -18 | SEQ ID NO: 74 |
| ATGTCT-------------------TGCTCGCGCTACTCTCTCT | -19 | SEQ ID NO: 75 |

| | | |
|---|---|---|
| Note: The "-" in the table is marked as "d" in the sequence listing. | | |

**Table D: Sequence changes produced from base 23016448 to 23016490 on chromosome 14 after the introduction of T3:**

| "-" means the deleted base after editing, "n" means the insertion of any base | Change in sequence length | Sequence numbers |
|---|---|---|
| TATCCAGAACCCTGACCCTGCCGTGTACCAGCTGAGAGACTCT | Original sequence | SEQ ID NO: 23 |
| TATCCAGAACCCTGACCCTGCnCGTGTACCAGCTGAGAGACTCT | 1 | SEQ ID NO: 76 |
| TATCCAGAACCCTGACCCT-----------GCTGAGAGACTCT | -11 | SEQ ID NO: 77 |
| TATCCAGAACCCTGACCCTG-----TACCAGCTGAGAGACTCT | -5 | SEQ ID NO: 78 |
| TATCCAGA------------------------TGAGAGACTCT | -24 | SEQ ID NO: 79 |
| TATCCAGAACCCTGACCCTG-CGTGTACCAGCTGAGAGACTCT | -1 | SEQ ID NO: 80 |
| TATCCAGAACCCTGAC------------CAGCTGAGAGACTCT | -12 | SEQ ID NO: 81 |
| TATCCAGAACCCTGACCCTGCnnCGTGTACCAGCTGAGAGACTCT | 2 | SEQ ID NO: 82 |
| TATCCAGAACCCTGA-----------ACCAGCTGAGAGACTCT | -11 | SEQ ID NO: 83 |
| TATCCAGAACCCTGACCCTG------------TGAGAGACTCT | -12 | SEQ ID NO: 84 |
| TATCCAGAACCCTGACC------TGTACCAGCTGAGAGACTCT | -6 | SEQ ID NO: 85 |
| TATCCA-----------------TGTACCAGCTGAGAGACTCT | -17 | SEQ ID NO: 86 |
| TATCCA------------------GTACCAGCTGAGAGACTCT | -18 | SEQ ID NO: 87 |
| TATCCAGAACCCTGACCCTGC--TGTACCAGCTGAGAGACTCT | -2 | SEQ ID NO: 88 |
| TATCCAGA--------------GTGTACCAGCTGAGAGACTCT | -14 | SEQ ID NO: 89 |
| TATCCAGAACCCTGACCCTG-GTGTACCAGCTGAGAGACTCT | -2 | SEQ ID NO: 90 |
| TATCCAGAACCCTGA--------------AGCTGAGAGACTCT | -14 | SEQ ID NO: 91 |
| TATCCAGAACCCTGA--------------------GAGACTCT | -20 | SEQ ID NO: 92 |
| TATCCA--------------------ACCAGCTGAGAGACTCT | -20 | SEQ ID NO: 93 |
| TATCCAGAACC------------TGTACCAGCTGAGAGACTCT | -12 | SEQ ID NO: 94 |
| TATCCAGAACCCTGACCCTG---TGTACCAGCTGAGAGACTCT | -3 | SEQ ID NO: 95 |
| TATCCAGAACCCTGA-------GTGTACCAGCTGAGAGACTCT | -7 | SEQ ID NO: 96 |
| TATCCAGA------------------ACCAGCTGAGAGACTCT | -18 | SEQ ID NO: 97 |
| TATCCAGAACCCTGACC-----GTGTACCAGCTGAGAGACTCT | -5 | SEQ ID NO: 98 |
| TATCCAGAACCCTGACCCTG--------------AGAGACTCT | -14 | SEQ ID NO: 99 |
| TATCCAGAACCCTG------------------TGAGAGACTCT | -18 | SEQ ID NO: 100 |
| TATCCAGAACCCTG-----------TACCAGCTGAGAGACTCT | -11 | SEQ ID NO: 101 |
| TATCCAGAACCCTGAC-------------AGCTGAGAGACTCT | -13 | SEQ ID NO: 102 |

| | | |
|---|---|---|
| Note: The "-" in the table is marked as "d" in the sequence listing. | | |

**Table E: Sequence changes produced from base 23016448 to 23016490 on chromosome 14 after the introduction of T2:**

| "-" means the deleted base after editing, "n" means the insertion of any base | Change in sequence length | Sequence numbers |
|---|---|---|
| TATCCAGAACCCTGACCCTGCCGTGTACCAGCTGAGAGACTCT | Original sequence | SEQ ID NO: 23 |
| TATCCAGAACCCTG------CCGTGTACCAGCTGAGAGACTCT | -6 | SEQ ID NO: 103 |
| TATCCAGAACCCTGA------CGTGTACCAGCTGAGAGACTCT | -6 | SEQ ID NO: 104 |
| TATCCAGAACCCTG-CCCTGCCGTGTACCAGCTGAGAGACTCT | -1 | SEQ ID NO: 105 |
| TATCCAGAACCCTGAnCCCTGCCGTGTACCAGCTGAGAGACTCT | 1 | SEQ ID NO: 106 |
| TATCCAGAACCCTG--CCTGCCGTGTACCAGCTGAGAGACTCT | -2 | SEQ ID NO: 107 |
| T------------------------TACCAGCTGAGAGACTCT | -24 | SEQ ID NO: 108 |
| TATCCAGAACCCTG----TGCCGTGTACCAGCTGAGAGACTCT | -4 | SEQ ID NO: 109 |
| TATCCA-----------------TGTACCAGCTGAGAGACTCT | -17 | SEQ ID NO: 110 |
| TATC-----------CCCTGCCGTGTACCAGCTGAGAGACTCT | -11 | SEQ ID NO: 111 |
| TATCCAGA------------------ACCAGCTGAGAGACTCT | -18 | SEQ ID NO: 112 |
| TATCC------------CTGCCGTGTACCAGCTGAGAGACTCT | -12 | SEQ ID NO: 113 |
| TATC-------------------------AGCTGAGAGACTCT | -25 | SEQ ID NO: 114 |
| TATCCAGA-------CCCTGCCGTGTACCAGCTGAGAGACTCT | -7 | SEQ ID NO: 115 |
| TATCCAGAACCCTG-------CGTGTACCAGCTGAGAGACTCT | -7 | SEQ ID NO: 116 |
| TATCCAGAAC-----CCCTGCCGTGTACCAGCTGAGAGACTCT | -5 | SEQ ID NO: 117 |
| T-----------------------GTACCAGCTGAGAGACTCT | -23 | SEQ ID NO: 118 |
| ---------------CCCTGCCGTGTACCAGCTGAGAGACTCT | -15 | SEQ ID NO: 119 |
| TATCCAGAACCCTGAnnnnnnnnnnnnnnnCCCTGCCGTGTACCAGCTGA GAGACTCT | 15 | SEQ ID NO: 120 |
| TATCCAGA-------------CGTGTACCAGCTGAGAGACTCT | -13 | SEQ ID NO: 121 |
| TATCCAGA----------TGCCGTGTACCAGCTGAGAGACTCT | -10 | SEQ ID NO: 122 |
| TATCC----------CCCTGCCGTGTACCAGCTGAGAGACTCT | -10 | SEQ ID NO: 123 |

| | | |
|---|---|---|
| Note: The "-" in the table is marked as "d" in the sequence listing. | | |

**Table F: Sequence changes produced from base 242800936 to 242800978 on chromosome 2 after the introduction of P2:**

| "-" means the deleted base after editing, "n" means the insertion of any base | Change in sequence length | Sequence numbers |
|---|---|---|
| AGCCCAGTTGTAGCACCGCCCAGACGACTGGCCAGGGCGCCTG | Original sequence | SEQ ID NO: 25 |
| AGCCCAGTTGTAGCACCGCCCnAGACGACTGGCCAGGGCGCCTG | 1 | SEQ ID NO: 124 |
| AGCCCAGTTGTAGCACCGCCCnnAGACGACTGGCCAGGGCGCCTG | 2 | SEQ ID NO: 125 |
| AGCCCAGTTGTAGCACCG-----ACGACTGGCCAGGGCGCCTG | -5 | SEQ ID NO: 126 |
| AGCCCAGTTGTAGCACCGCCCnnnnnnnnnnnnnnAGACGACTGGCCAGG | 14 | SEQ ID NO: 127 |
| AGCCCAGTTGTA-----------------GGCCAGGGCGCCTG | -17 | SEQ ID NO: 128 |
| AGCCCAGTT------------------------AGGGCGCCTG | -24 | SEQ ID NO: 129 |
| AGCCCA----------------------TGGCCAGGGCGCCTG | -22 | SEQ ID NO: 130 |
| AGCCCAGTTGTAGCACCGCC-AGACGACTGGCCAGGGCGCCTG | -1 | SEQ ID NO: 131 |
| AGCCCAGTTGTAGCACCGCCC-----ACTGGCCAGGGCGCCTG | -5 | SEQ ID NO: 132 |
| AGCCCAGTT---------------CGACTGGCCAGGGCGCCTG | -15 | SEQ ID NO: 133 |
| AGCCCAGTTGTAGCACCGCCC-ACGACTGGCCAGGGCGCCTG | -2 | SEQ ID NO: 134 |
| AGCCCAGTTGTAGCACCGC-AGACGACTGGCCAGGGCGCCTG | -2 | SEQ ID NO: 135 |
| AGCCCAGTTGTAG---------GACGACTGGCCAGGGCGCCTG | -9 | SEQ ID NO: 136 |
| AGCCCAGTTGTAG----------ACGACTGGCCAGGGCGCCTG | -10 | SEQ ID NO: 137 |
| AGCCCAGT------------------------CAGGGCGCCTG | -24 | SEQ ID NO: 138 |
| AGCCCA------------------------------GCGCCTG | -30 | SEQ ID NO: 139 |
| AGCCCAGTTGTAGCACCG----GACGACTGGCCAGGGCGCCTG | -4 | SEQ ID NO: 140 |
| AGCCCAGTTGTAGCACCGCC-GACGACTGGCCAGGGCGCCTG | -2 | SEQ ID NO: 141 |
| AGCCCAGTTG---------------GACTGGCCAGGGCGCCTG | -15 | SEQ ID NO: 142 |
| AGCCCAGTTGT-----------------------GGGCGCCTG | -23 | SEQ ID NO: 143 |
| AGCCCA---------------AGACGACTGGCCAGGGCGCCTG | -15 | SEQ ID NO: 144 |
| AGCCCAGTTGT---------------ACTGGCCAGGGCGCCTG | -15 | SEQ ID NO: 145 |
| AGCCCA---------------------CTGGCCAGGGCGCCTG | -21 | SEQ ID NO: 146 |

| | | |
|---|---|---|
| Note: The "-" in the table is marked as "d" in the sequence listing. | | |

**Table G: Sequence changes produced from positions 242795009 to 242795051 on chromosome 2 after the introduction of P1.**

| "-" means the deleted base after editing, "n" means the insertion of any base | Change in sequence length | Sequence numbers |
|---|---|---|
| CAGTTTAGCACGAAGCTCTCCGATGTGTTGGAGAAGCTGCAGG | Original sequence | SEQ ID NO: 26 |
| CAGTTTAGCACGAAGCTCTCCGATGnTGTTGGAGAAGCTGCAGG | 1 | SEQ ID NO: 147 |
| CAGTTTAGCACGAAGCTCTCCGATG--TTGGAGAAGCTGCAGG | -2 | SEQ ID NO: 148 |
| CAGTTTAGCACGAAGCTCTCCGAT-TGTTGGAGAAGCTGCAGG | -1 | SEQ ID NO: 149 |
| CAGTTT AGCACGAAGCTCTCCG---TGTTGGAGAAGCTGCAGG | -3 | SEQ ID NO: 150 |
| CAGTTTAGCAC--------------TGTTGGAGAAGCTGCAGG | -14 | SEQ ID NO: 151 |
| CAGTTTAGCACGAAGCTCTC-----TGTTGGAGAAGCTGCAGG | -5 | SEQ ID NO: 152 |
| CAGTTTAGCACGAAGCTCTCC----TGTTGGAGAAGCTGCAGG | -4 | SEQ ID NO: 153 |
| CAGTTTAGCA---------------------AGAAGCTGCAGG | -21 | SEQ ID NO: 154 |
| CAGTTTAGCACGAAGCT--------TGTTGGAGAAGCTGCAGG | -8 | SEQ ID NO: 155 |
| CAGTTTAGCACGAAGCTCT------TGTTGGAGAAGCTGCAGG | -6 | SEQ ID NO: 156 |
| CAGTTTAGCACGAAGCTCTCCGATGnnnnnnnnnnnnTGTTGGAGA AGCTGCAGG | 13 | SEQ ID NO: 157 |
| CAGTTTAGCA-----------------GTTGGAGAAGCTGCAGG | -16 | SEQ ID NO: 158 |
| CAGTTTAGCACGAAGCTCTCCGATGnnTGTTGGAGAAGCTGCAG G | 2 | SEQ ID NO: 159 |
| CAGTTTAGCA-----------------TTGGAGAAGCTGCAGG | -17 | SEQ ID NO: 160 |
| CAGTTTAGCACG-------------TGTTGGAGAAGCTGCAGG | -13 | SEQ ID NO: 161 |
| CAGTTTAGCACGAAGCTCT-------GTTGGAGAAGCTGCAGG | -7 | SEQ ID NO: 162 |
| CAGTTTAGCA---------------TGTTGGAGAAGCTGCAGG | -15 | SEQ ID NO: 163 |
| CAGTTTAGCACGAAGCTCTCCGATGnnnnnnnnTGTTGGAGAAGCT GCAGG | 8 | SEQ ID NO: 164 |

| | | |
|---|---|---|
| Note: The "-" in the table is marked as "d" in the sequence listing. | | |

3.6 At the same time, the potential off-target loci on human whole genome for TRAC-sg3 (T2, T3), B2M-sg2 (B2, B3), and PD-1-sg2 (P1, P2) are predicted. the predicted off-target regions possibly affecting the expression of other genes are amplified and analyzed in order to confirm that the knockout of TRAC, B2M, and PD-1 at the molecular level does not introduce the off-target gene knockout non-specifically. The results are shown in Table 3 and Figure 5.

**Table 3: Results of off-target analysis of TRAC, TRAC/B2M (DKO), and TRAC/B2M/PD-1 (TKO) knockout T cells**

| sgRNA | Potential off-target loci | INDEL (%) |
|---|---|---|
| | chrX:73096741-73096783 | 0 |
| | chr15:45003596-45003638 | 0 |
| | chr7:97861900-97861942 | 0 |
| | chr2:161504884-161504926 | 0 |
| | chr7:147683672-147683714 | 0 |
| | chr18:42654204-42654246 | 0 |
| T2, T3 | chr8:10627233-10627275 | 0 |
| | chr1:151805238-151805280 | 0 |
| | chr16:85219576-85219618 | 0 |
| | chr10:31873265-31873307 | 0 |
| | chr12:122582230-122582272 | 0 |
| | chr10:134549611-134549653 | 0 |
| | chr22:21970051-21970093 | 0 |
| | **chr14:23016448-23016490** | 81 |
| | chr15:40533648-40533690 | 0 |
| | chrY:23456283-23456325 | 0 |
| | chr15:24604012-24604054 | 0 |
| B2, B3 | chr19:5105831-5105873 | 0 |
| | chr13:31092400-31092442 | 0 |
| | chr19:13194699-13194741 | 0 |
| | chr4:137589707-137589749 | 0 |
| | chr5:604003-604045 | 0 |
| | chr20:7173027-7173069 | 0 |
| | **chr15:45003745-45003788** | 79 |
| | chr9:85813380-85813422 | 0 |
| | chr14:102977368-102977410 | 0 |
| | chr2:222944815-222944857 | 0 |
| | chr15:82710483-82710525 | 0 |
| | chr19:1936279-1936321 | 0 |
| | chr2:242557082-242557124 | 0 |
| | chrY:26302188-26302230 | 0 |
| | chr19:41105118-41105160 | 0 |
| | chr4:96091335-96091377 | 0 |
| | chr15:76181855-76181897 | 0 |
| P1, P2 | chr11:73202191-73202233 | 0 |
| | chr9:135490161-135490203 | 0 |
| | chr9:126151505-126151547 | 0 |
| | chr5:133205312-133205354 | 0 |
| | chr3:73161817-73161859 | 0 |
| | chr17:2771461-2771503 | 0 |
| | chr7:1048123-1048165 | 0 |
| | chr9:112168511-112168553 | 0 |
| | chrY:27660208-27660250 | 0 |
| | chr17:70138831-70138873 | 0 |
| | chr2:99390127-99390169 | 0 |
| | **chr2:242800936-242800978** | 76 |

| | | |
|---|---|---|
| Note: All the above sequence locus information is determined according to the reference database: GRCh37 (hg19). | | |

It can be seen from Table 3 that the genes of TRAC, B2M and PD-1 do not undergo any off-target, indicating that the system meets the requirement on specificity for genome editing.

The above results show that by using TRAC-sg2 (T2), TRAC-sg3 (T3); B2M-sg2 (B2), B2M-sg3 (B3), and PD-1-sg1 (P1), PD-1-sg2 (P2) as sgRNA to modify the T cells with genome editing technology, the TRAC, B2M, and PD-1 genes in the universal T cells obtained by further screening are completely knocked out, and no gene mutation is found at the potential off-target loci.

### 4. Amplification of the genetically modified T cells

The sorted T cells are activated by using cytokine, and then the cell density is adjusted to 1×10⁶ cells/mL with a T cells culture medium. After 72 hrs., the state of the cells is observed, and the cell suspension is collected and centrifuged at 300g for 7 min. The supernatant is discarded, and the pellet was washed twice with DPBS (Gibco), and then the cell density is adjusted to 2.5 X 10⁷ cells/mL with a medium containing electroporation reagent. The Cas9 mRNA prepared by HISCRIBE™ T7 ARCA mRNA Kit (manufacturer: NEB, Cat. No.: cat#E2060S) and synthetic sgRNA are mixed and adjusted to a final concentration of 2.5×10⁶ cells and 8 µg RNA per 100 µl (4 µg each of Cas9 mRNA and sgRNA). The cells are cultured after the RNAs are introduced inside by a BTX Agile pulse MAX electroporator. The growth of the cells is observed every day. The cells are counted and the medium are replaced every other day. Phenotypic analysis on the genetically modified T cells is performed. As shown in Fig. 3, the efficiency of TCR knockout alone is about 90.42%, the efficiency of TCR and B2M knockout (DKO) is about 81.39%, and the efficiency of simultaneous knockout of three genes (TKO) is as high as 67.91%. After culturing for 8 days, the resulting T cells are subjected to quality control monitoring.

### 5. Detection of the efficiency of genome editing with CRISPR/Cas9

Samples are taken from the cells cultured for 8 days, and the human genome extraction kit (manufacturer: TIAN GEN, Cat. No.: cat#DP304-03) is used to extract the genome from the samples. Meanwhile the corresponding sequencing primers are designed, and the target fragments are prepared by PCR technology. The target fragment are sequenced with the corresponding primers using Sanger sequencing. The sequencing results are analyzed by using TIDE software, and the results are shown in Figures 2A, 2C, and 2E. The optimized sgRNAs can efficiently knock out the corresponding genes.

### 6. Screening of target cells

TCR and/or B2M and/or PD-1 negative, CD4 and CD8 positive T cells are screened out by the following methods.

TCR and/or B2M and/or PD-1 negative, CD4 and CD8 positive T cells are screened out by immunomagnetic beads technique, and the status of the edited T cells is monitored through the observation of the T cells viability.

In the first step, the T cells are collected on the 12th-14th day after electroporation, by centrifuging at 400G for 5 min, the supernatant is discarded. The cells are suspended into 1×10⁸/ml with Easy buffer, and then transferred to a 5 ml flow cytometry tube. The cells still expressing TCR, B2M, and PD-1 in the T cells are removed with a screening reagent, and after the screening the final product (i.e., universal T cells) is obtained.

In the second step, a small number of T cells are taken for test by flow cytometry, and TCR and/or B2M and/or PD-1 cell surface biomarkers are stained. Once TCR and/or B2M and/or PD-1 positive rate is < 1%, the next step is continued. In this example, the TCR positive rate is 1%, the positive rate of TCR and B2M DKO T cells is <0.79%, and the positive rate of TCR, B2M and PD-1 TKO T cells is <1%, as shown in FIG. 4. The viability of the purified T cells is not significantly affected as each of them is above 85%, as shown in Table 4.

**Table 4: Viability of the T cells with knockout of TCR, TCR/B2M (DKO) and TCR/B2M/PD-1 (TKO) after screening and purification**

| | Viability (%) | | | |
|---|---|---|---|---|
| | | | | |
| Days | T cells | T3 | DKO | TKO |
| 6 | 92.88 | 81.71 | 82.4 | 83 |
| 9 | 92.98 | 90.42 | 87.66 | 91.77 |
| 11 | 93.27 | 92.98 | 89.62 | 88.42 |

### 7. Off-target analysis

7.1. 1×10⁶ T cells are collected, and the genome is extracted (manufacturer: QIAGEN, Cat. No.: Cat#69504).
7.2. The extracted genome is subjected to Cas9 digestion *in vitro*
The *in vitro* Cas9 digestion reaction system is as follows:

| Reagents | Volumes (µl) |
|---|---|
| Nuclease-free water | 20.0 |
| 10 × Cas9 nucleic acid reaction buffer | 3.0 |
| sgRNA (2000 ng/ul) | 1.5 |
| Cas9 nuclease (1 uM) | 1.0 |
| Pre-incubation, 25°C, 10 min | |
| Genomic DNA (1600 ng/ul) | 4.5 |

7.3. Gently pipetting up and down to mix, and incubating at 37°C for 15 min;
7.4. Adding 1µl of proteinase K (manufacturer: Tiangen, Cat. No.: Cat# RT403), mixing gently, and incubating for 10 min at room temperature;
7.5. 5µl of sample is taken for gel electrophoresis to detect the effect of *in vitro* Cas9 digestion reaction;
7.6. The remaining sample is subjected to human whole genome sequencing.

The results are shown in Fig. 5, no off-target phenomenon is observed.

### Example 2: Preparation of universal T cells

### Preparation and amplification of universal CAR-T cells

The sorted T cells are activated by using cytokine, and then the cell density is adjusted to 1×10⁶ cells/mL with a medium for culturing T cells. After 72 hrs., the state of the cells is observed, and the cell suspension is collected, centrifuging at 300g for 7 min, the supernatant is discarded, washing twice with DPBS solution (manufacturer: Gibco; Cat. No. 1924294), and then the cell density is adjusted to 2.5 X 10⁷ cells/mL with a medium containing electroporation reagent. The Cas9 mRNA prepared by HISCRIBE™ T7 ARCA mRNA Kit (tailed) (manufacturer: NEB, Cat. No.: cat#E2060S) and synthetic sgRNA are used to mix the T cells and RNA to a final concentration of 2.5×10⁶ cells and 8 µg RNA per 100 µl (4 µg each of Cas9 mRNA and sgRNA), and then RNA is introduced into the cells by a BTX Agile pulse MAX electroporator for culturing. The growth of the cells is observed every day, the cells are counted and the fluid are replaced every other day, and on the 5th day a lentivirus packaged with CAR (including anti-CD19 scFv, linker, CD8 alpha hinge, CD8 transmembrane domain, 4-11BB signaling domain and CD3 zeta, see US20140271635A1 for the specific structure) is added according to the ratio of MOI=2-10.

### Screening of universal CAR-T cells

TCR and/or B2M and/or PD-1 negative, CD4 and CD8 positive T cells are screened by the following methods.

TCR and/or B2M and/or PD-1 negative, CD4 and CD8 positive T cells are screened by immunomagnetic beads technique, and status of the edited T cells is monitored by T cell viability. The specific steps are as follows:
In the first step, the T cells after electroporation are collected on the 12th-14th day, centrifuging at 400G for 5 min, the supernatant is discarded, the cells are dissolved into 1×10⁸/ml with Easy buffer, and then the cells are transferred to a 5 ml flow tube. The cells still expressing TCR, B2M, and PD-1 in the T cells are removed by a screening magnetic beads, and the final product (i.e., universal T cells) is obtained by screening.

In the second step, a small number of T cells are taken for flow cytometry detection, and TCR and/or B2M and/or PD-1 cell surface biomarkers are stained, if TCR and/or B2M and/or PD-1 positive rate is < 1%, the next step is continued. In this example, the TCR positive rate is 1%, the positive rate of T cells of TCR and B2M DKO is <0.79%, and the positive rate of T cells of TCR, B2M and PD-1 TKO is <1%, as shown in FIGs. 5 and 6. The viability of the purified T cells is not significantly affected, each of them is above 85%.

**Table 5:**

| | Viability (%) | | | | |
|---|---|---|---|---|---|
| Days | T cells | CAR-T | TCR^{neg} CAR-T | DKO CAR-T | TKO CAR-T |
| 7 | 94.05 | 94.05 | 89.19 | 85.33 | 83.04 |
| 10 | 97.22 | 97.12 | 97.09 | 96.97 | 96.43 |
| 14 | 93.87 | 96.25 | 96.88 | 95.32 | 94.91 |
| 15 | 95.91 | 95.14 | 96.71 | 95.34 | 94.95 |
| 17 | 90.19 | 90.42 | 95.3 | 91.58 | 91.52 |

### Example 3: Verification of the function of universal CAR-T

The killing efficacy of the universal CAR-T cells (i.e., effector cells) obtained in Example 2 on B cell type acute lymphoblastic leukemia cells is observed.

### A. In vitro killing efficacy on specific tumor cells

The experimental steps according to the present invention are as follows:
The first step: labelling the target cells
   The target cells (human Burkitt's lymphoma cells Raji, K562; all the cells are from ATCC) are labeled by using the CELL TRACE™ Far Red Cell Proliferation Kit (manufacturer: Gibco; Cat. No. 1888569).
   1. Diluting the Cell Trace™ Far Red Cell Proliferation into 1 mmol solution with double distilled water;
   2. Taking 1×10⁶ target cells to centrifuge at 400g for 5 min, then removing the supernatant;
   3. Adding 1 µl of Cell Trace™ Far Red Cell Proliferation solution, and incubating at 37°C for 20 min in the dark;
   4. Adding the cells into a medium for culturing T cells, and incubating at 37°C for 5 min;
   5. Centrifuging at 400g for 5 min, then removing the supernatant, and the labeling is completed.
The second step: detection of the killing efficacy of effector cells on target cells
   The labeled target cells are re-suspended with R1640+10% FBS medium at a density of 2×10⁵ cells/ml. Take 500 µl of the cells to add to a 48-well plate. According to the appropriate effector-to-target ratio (2.5:1; 1.25:1; 0.6:1), 500 µl of effector cells are added to each well, and T cells and healthy human cord blood CAR-T cells (on the second day of culturing the T cells, adding a lentivirus packed with CAR (see US20140271635A1 for the specific structure) at a ratio of MOI=2-10 to prepare the CAR-T) are used as control cells, and each group has 3 parallels, and a separate target cell group is designed to detect the mortality rate. After incubating at 37°C, 5% CO₂ for 12-16 hrs., centrifuging at 400 g for 5 min, the cell pellet is re-suspended with 150 µl of DPBS (manufacturer: Gibco; Cat. No. 1924294). After staining with PI (manufacturer: Sigma; Cat. No. P4170), the mortality rate of the target cells is detected by flow cytometry. The results are shown in Fig. 7 that the killing efficacy of the universal CAR-T on specific target cells is substantively consistent with other CAR-T cells, and the efficacy are superior to that of the T cells. Meanwhile, there is almost no killing efficacy on non-specific target cells.
The third step: ELISA test for cytokine release
   The labeled target cells are re-suspended with R1640+10% FBS medium at a density of 2×10⁵ cells/mL, and 500 µl of the cells are add to a 48-well plate. According to the appropriate effector-to-target ratio (10:1), 500 µl of effector cells are added to each well, and T cells and healthy human cord blood CAR-T cells are used as control cells, and each group has 3 parallels, and a separate target cell group is designed. After incubating at 37°C, 5% CO₂ for 12-16 hours, taking 100 µl of the cultural supernatant to centrifuge at 400 g for 5 min and discarding the sediment, the resulting supernatant is used for the test of the cytokine release by using LEGEND MAX™ Human IL-2/IFN-gama (manufacturer: Biolegend; Cat. No.: 431807, 430108) Kit according to the instructions.

The results are shown in Fig. 8. It shows that the killing efficacy of universal CAR-T against Raji cells is substantively equal to or slightly better than that of the common CAR-T, especially the IFN- γ release rate of TCR^{neg} CAR-T is much higher than that of the common CAR-T. And both of them have lower lethality to K562, therefore the cytokine release is relatively less.

### B. In vivo killing efficacy of the universal CAR-T on the specific tumor cells

### 1. Cell line: human lymphoma cell line

Raji^{tg(luciferase-GFP)}/Bcgen cell is human Burkitt's lymphoma cell line with positive CD19 expression, and can serve as a target cell for CAR-T cell. Raji^{luc-GFP} is modified to express both GFP and luciferase. A human lymphoma model of mouse can be constructed by tail intravenous injection. The tumor formation area is measured by calculating the fluorescence it presents by using XenoLight D-Luciferin, Potassium Salt (manufacturer: PerkinElmer; Cat. No. 122799) in combination with a small animal living imager.

### 2. Raji^{tg(luciferase-GFP)}/Bcgen cell culture

The Raji^{tg(luciferase-GFP)}/Bcgen cell line is a suspension cell line capable of growing rapidly in RPMI 1640 medium containing 10% FBS. Passaging is required when the cell density is 2-3 × 10⁶/ml. When passaging, the cell suspension is taken to a centrifuge tube. After centrifuging at 300 g for 6 min, the supernatant is discarded. The cell density is adjusted to 1×10⁶ cells/ml for subsequent culturing. In normal growth conditions, the cells are passaged every other day, and cell density can be maintained between 0.8-1 × 10⁶ cells/ml.

### 3. Mouse modeling

Each of the twenty-five NPT female mice of 7-10 week-old is injected with 5× 10⁵ tumor cells (Raji^{tg(luciferase-GFP)}/Bcgen) in a single tail intravenous injection. They are weighed every other day and observed once a day. 3-5 days after inoculating the tumor cells, the tumor formation area and tumor enrichment is presented by XenoLight D-Luciferin, Potassium Salt (manufacturer: PerkinElmer; Cat. No. 122799) in combination with the small animal living imager. It is taken as an indicator for dividing the mice randomly into 6 groups: saline group, T cell group, CAR-T cell group, TCR^{neg}-CAR-T group, DKO-CAR-T group, and TKO-CAR-T group.

### 4. Administration of the Mouse lymphoma model

The day of modeling is recorded as D0. 200 µl of normal saline, 200 µl of human T cells (total 2×10⁶ cells/mouse), 200 µl of CAR-T cells (total 2×10⁶/mouse), 200 µl of TCR^{neg}-CAR-T cells (total 2×10⁶/mouse), 200 µl of DKO-CAR-T cells (total 2×10⁶/mouse), and 200 µl of TKO-CAR-T cells (total 2×10⁶/mouse) are injected tail-intravenous. Each of the mice is administered with a single dose. The results are shown in FIGs. 9A-9B and FIG. 10. It can be seen that the universal CAR-T provided by the present invention is excellent in inhibiting and killing tumor cells at earlier stage, and is almost consistent with that of CAR-T cells; and at the later stage said universal CAR-T has a significant inhibitory effect on tumor cells than that of the ordinary CAR-T.

### 5. Monitoring the mice after administration

The mice are monitored every day after administration. It includes body weight, skin integrity, hair, mental state, motion level, and motor coordination. The body weight of each mice is recorded every 2 days for 37 days. The elimination of tumor area and the reduction of tumor enrichment are used as indicators to evaluate the function of effector cells, and the safety of universal CAR-T is estimated according to the skin integrity, hair, mental state, motion level and motor coordination. As shown in Figure 11, the body weights of the mice are not decreased, and their skin and hair are intact. They are in a good spirit. Their movements are coordinated, and no GVHD response occurred.

## Claims

1. A method for preparing a genetically modified T cell, comprising: disrupting the following genomic regions in the T cell by genome editing technology:
(i) the TRAC genomic region from base 23016448 to 23016490 on chromosome 14;
(ii) the B2M genomic region from base 45003745 to 45003788 on chromosome 15; and/or
(iii) the PD-1 genomic region from base 242800936 to 242800978 on chromosome 2, or the PD-1 genomic region from base 242795009 to 242795051 on chromosome 2.

2. The method of claim 1, wherein all the TRAC genomic region, the B2M genomic region and the PD-1 genomic region are edited.

3. The method of claim 1 or 2, wherein the genome editing technology is a zinc finger nuclease-based genome editing technology, a TALEN genome editing technology, or a CRISPR/Cas genome editing technology.

4. The method of claim 3, wherein the genome editing technology is a CRISPR/Cas9 genome editing technology.

5. The method of any one of claims 1-4, wherein:
(i) the target nucleotide sequence of the TRAC gene is complementary to a sequence selected from any one of SEQ ID NOs: 2-5;
(ii) the target nucleotide sequence of the B2M gene is complementary to a sequence selected from any one of SEQ ID NOs: 6-13; and/or
(iii) The target nucleotide sequence of the PD-1 gene is complementary to a sequence selected from any one of SEQ ID NOs: 14-22.

6. The method of claim 4, wherein:
(i) introducing a sgRNA comprising a sequence targeting the TRAC gene into the T cell for editing the TRAC genomic region;
(ii) introducing a sgRNA comprising a sequence targeting the B2M gene into the T cell for editing the B2M genomic region; and/or
(iii) introducing a sgRNA comprising a sequence targeting the PD-1 gene into the T cell for editing the PD-1 genomic region.

7. The method of claim 6, comprising:
(i) introducing a sgRNA comprising a sequence selected from any one of SEQ ID NOs: 2-5 into the T cell for editing the TRAC genomic region;
(ii) introducing a sgRNA comprising a sequence selected from any one of SEQ ID NOs: 6-14 into the T cell for editing the B2M genomic region; and/or
(iii) introducing a sgRNA comprising a sequence selected from any one of SEQ ID NOs: 15-22 into the T cell for editing the PD-1 genomic region.

8. The method of claim 7 or 8, comprising simultaneously introducing the TRAC-targeted sgRNA, the B2M-targeted sgRNA, and the PD-1-targeted sgRNA into the T cell.

9. The method of any one of claims 6-8, wherein the sgRNA is a 2'-O-methyl analog sgRNA and/or internucleotide 3'-thio sgRNA.

10. The method of claim 9, wherein the sgRNA has 2'-O-methyl nucleotide analogs on the first one, two, and/or three base(s) of the 5' end and/or the last base of the 3' end.

11. The method of any one of claims 6-10, wherein the sgRNA and a Cas9-encoding nucleotide sequence are co-introduced into the T cell.

12. The method of claim 11, wherein the sgRNA and a Cas9-encoding nucleotide sequence are co-introduced into the T cell by electroporation, preferably the electroporation condition comprises any one selected from the group consisting of: 150-250V, 0.5-2ms; 150V, 2ms; 160V, 2ms; 170V, 2ms; 180V, 2ms; 190V, 1ms; 200V, 1ms; 210V, 1ms; 220V, 1ms; 230V, 1ms; 240V, 1ms; and 250V, 0.5ms.

13. The method of any one of claims 1-12, further comprising screening out a T cell having low expression level of TRAC, B2M and/or PD-1 from genetically modified T cells.

14. The method of any one of claims 1-13, wherein the efficiency of knockout of the TRAC, B2M or PD-1 gene is 90% or more; the efficiency of simultaneous knockout of the TRAC and B2M genes is 75% or more; or the efficiency of simultaneous knockout of the TRAC, B2M and PD-1 genes is 65% or more.

15. The method of any one of claims 1-14, wherein the T cells are derived from a healthy subject.

16. A genetically modified T cell prepared by the method of any one of claims 1-15.

17. A genetically modified T cell, wherein in the T cell:
(i) one or more loci in the TRAC genomic region from base 23016448 to 23016490 on chromosome 14 are disrupted by genome editing technology;
(ii) one or more loci in the B2M genomic region from base 45003745 to 45003788 on chromosome 15 are disrupted by genome editing technology; and/or
(iii) one or more loci in the PD-1 genomic region from base 242800936 to 242800978 on chromosome 2, or the PD-1 genomic region from base 242795009 to 242795051 on chromosome 2 are disrupted by genome editing technology.

18. Use of the genetically modified T cell of claim 16 or 17 for the preparation of a T cell for adoptive cellular therapy.

19. The use of claim 18, wherein the T cell for adoptive cellular therapy is a CAR-T cell or a TCR-T cell.

20. A method for preparing a CAR-T cell or a TCR-T cell, comprising: introducing a chimeric antigen receptor (CAR) or the nucleotides encoding the chimeric antigen receptor, or an engineered T cell receptor (TCR) or the nucleotides encoding the T cell receptor into the genetically modified T cell of claim 16 or 17.

21. A method for preparing a CAR-T cell or a TCR-T cell, comprising:
(i) introducing a sgRNA comprising a sequence targeting the TRAC gene from base 23016448 to 23016490 on chromosome 14 into the T cell to disrupt the TRAC genomic region; and/or
(ii) introducing a sgRNA comprising a sequence targeting the B2M genomic region from base 45003745 to 45003788 on chromosome 15 into the T cell to disrupt the B2M genomic region; and/or
(iii) introducing a sgRNA comprising a sequence targeting the PD-1 genomic region from base 242800936 to 242800978 on chromosome 2, or the PD-1 genomic region from base 242795009 to 242795051 to the T cell to disrupt the PD-1 genomic region; and
(iv) introducing a chimeric antigen receptor (CAR) or the nucleotides encoding the chimeric antigen receptor, or an engineered T cell receptor (TCR) or the nucleotides encoding the T cell receptor into the T cell.

22. The method of claim 21, wherein the TRAC-targeted sgRNA, the B2M-targeted sgRNA, and/or the PD-1-targeted sgRNA, and CAR or its encoding nucleotide sequence, or an engineered T cell receptor (TCR) or the nucleotides encoding the T cell receptor are simultaneously introduced into the T cell.

23. The method of claim 21, wherein introducing the CAR or its encoding nucleotide sequence, or an engineered T cell receptor (TCR) or the nucleotides encoding the T cell receptor into the T cell, prior to introducing the TRAC-targeted sgRNA, the B2M-targeted sgRNA, and/or the PD-1-targeted sgRNA into it; or introducing the CAR or its encoding nucleotide sequence, or an engineered T cell receptor (TCR) or the nucleotides encoding the T cell receptor into the T cell, after introducing the TRAC-targeted sgRNA, the B2M-targeted sgRNA, and/or the PD-1-targeted sgRNA into it.

24. The method of any one of claims 20-23, wherein the CAR-T is a universal CAR-T; or wherein the TCR-T is a universal TCR-T.

25. A CAR-T cell or TCR-T cell prepared by the method of any one of claims 20-24.

26. A CAR-T cell, comprising the genetically modified T cell of claim 16 or 17 which expresses a chimeric antigen receptor (CAR).

27. A TCR-T cell, comprising the genetically modified T cell of claim 16 or 17 which expresses an engineered TCR.

28. A composition, comprising the genetically modified T cell of claim 16 or 17, the CAR-T cell of claim 25 or 26, or the TCR-T cell of claim 25 or 27.

29. A method for treating a disease in a subject, comprising administering to the subject an effective amount of the CAR-T cell of claim 25 or 26, or the TCR-T cell of claim 25 or 27.

30. The method of claim 29, wherein the disease is a tumor.

31. The method of claim 30, wherein the tumor is a hematological tumor.

32. The method of claim 31, wherein the tumor is lymphoma or leukemia.

33. The method of any one of claims 29-32, wherein the CAR targets to CD19.

34. The method of any one of claims 29-33, wherein the T cell is not obtained from a subject.

35. A sgRNA comprising any one of SEQ ID NOs: 2-22.

36. The sgRNA of claim 35 is a 2'-O-methyl sgRNA analog and/or an internucleotide 3'-thio sgRNA modification.

37. The sgRNA of claim 36 has 2'-O-methyl nucleoside analogs on the first one, two, and/or three base(s) of the 5' end and/or the last base of the 3' end.
